# FASCICULE DE BREVET EUROPEEN

(11) **EP 1 913 021 B1**
(45) Date de publication et mention de la délivrance du brevet: **08.08.2012**
(21) Numéro de dépôt: 06778881.0
(22) Date de dépôt: 18.07.2006
(51) Int. Cl.: C07K 14/35, C12N 9/02, C12N 9/04, C12N 15/63, C12N 1/20, C12Q 1/68, C12N 15/11, C02F 3/34

(54) **POLYPEPTIDES AYANT UNE ACTIVITE DANS LA VOIE DE DEGRADATION DU MTBE ET LEURS UTILISATIONS**
POLYPEPTIDE MIT WIRKUNG AUF DEN MTBE-ABBAUWEG UND VERWENDUNG DAFÜR
POLYPEPTIDES HAVING AN ACTIVITY IN THE MTBE DEGRADATION PATH AND USES THEREOF

(30) Priorité: 18.07.2005 FR 0507577
(43) Date de publication de la demande: 23.04.2008
(73) Titulaire: Institut Français du Pétrole, 92852 Rueil Malmaison Cedex (FR); Conseil National de Recherches du Canada, Ottawa, ON K1A OR6 (CA)
(72) Inventeur: LOPES FERREIRA, Nicolas, F-08450 Raucourt (FR); LABBE, Diane, Pierrefonds, Quebec H8Z 2V5 (CA); MACIEL, Héléna, P-4200-320 Porto (PT); FAYOLLE-GUICHARD, Françoise, F-92140 Clamart (FR); MONOT, Frédéric, F-92000 Nanterre (FR); GREER, Charles W., Pointe Claire, Quebec H9R 2Y5 (CA)
(74) Mandataire: Cabinet Plasseraud
(86) Numéro de dépôt international: PCT/FR2006/001758
(87) Numéro de publication internationale: WO 2007/010133

(56) Documents cités:
- FRANCOIS ALAN ET AL: "Biodegradation of methyl tert-butyl ether and other fuel oxygenates by a new strain, Mycobacterium austroafricanum IFP 2012" APPLIED AND ENVIRONMENTAL MICROBIOLOGY, vol. 68, no. 6, juin 2002 (2002-06), pages 2754-2762, XP002379946 ISSN: 0099-2240
- DATABASE Geneseq [Online] 8 mai 2003 (2003-05-08), "Benzodiazepines biosynthesis gene SEQ ID No 1." XP002380298 extrait de EBI accession no. GSN:ABT32129 Database accession no. ABT32129
- DATABASE EMBL [Online] 5 octobre 2002 (2002-10-05), "Sequence 204 from Patent WO02052044." XP002424229 extrait de EBI accession no. EMBL:AX514006 Database accession no. AX514006
- DATABASE Geneseq [Online] 29 juillet 2004 (2004-07-29), "Klebsiella pneumoniae polynucleotide seqid 5495." XP002424230 extrait de EBI accession no. GSN:ACH99700 Database accession no. ACH99700
- DATABASE Geneseq [Online] 18 décembre 2003 (2003-12-18), "NOV protein-related forward PCR primer SEQ ID 325." XP002424231 extrait de EBI accession no. GSN:ADC26500 Database accession no. ADC26500
- DATABASE EMBL [Online] 9 mars 1995 (1995-03-09), "Oryza sativa (japonica cultivar-group) cDNA, partial sequence (S13004_4A)." XP002424232 extrait de EBI accession no. EMBL:D47478 Database accession no. D47478
- DATABASE Geneseq [Online] 4 mai 2001 (2001-05-04), "DNA analysis method PCR primer universal C beacon." XP002424233 extrait de EBI accession no. GSN:AAF74243 Database accession no. AAF74243
- DATABASE Geneseq [Online] 20 juin 2003 (2003-06-20), "Halohydrin dehydrogenase gene." XP002380297 extrait de EBI accession no. GSN:ACC00311 Database accession no. ACC00311
- DATABASE Geneseq [Online] 21 octobre 2004 (2004-10-21), "Primer of the invention #135." XP002424234 extrait de EBI accession no. GSN:ADQ90115 Database accession no. ADQ90115
- DATABASE EMBL [Online] 6 février 2005 (2005-02-06), "81490rsicen_8503.y1 Oryza sativa cv. LYP9 booting whole plant cDNA library Oryza sativa (indica cultivar-group) cDNA 5', mRNA sequence." XP002424235 extrait de EBI accession no. EMBL:CK077779 Database accession no. CK077779
- DATABASE EMBL [Online] 1 octobre 1998 (1998-10-01), "vz90h06.r1 Soares 2NbMT Mus musculus cDNA clone IMAGE:1344539 5' similar to TR:P97301 P97301 STROMAL CELL DERIVED FACTOR RECEPTOR 2 ;, mRNA sequence." XP002424236 extrait de EBI accession no. EMBL:AI153789 Database accession no. AI153789
- DATABASE Geneseq [Online] 24 janvier 2002 (2002-01-24), "Human SNP oligonucleotide #7647." XP002424237 extrait de EBI accession no. GSN:AAL34439 Database accession no. AAL34439
- DATABASE EMBL [Online] 22 septembre 2004 (2004-09-22), "OsIRUA004936 Oryza sativa Express Library Oryza sativa (indica cultivar-group) genomic, genomic survey sequence." XP002424238 extrait de EBI accession no. EMBL:CL956754 Database accession no. CL956754
- DATABASE EMBL [Online] 13 janvier 2003 (2003-01-13), "Rhodococcus sp. Phi2 cyclohexanone oxidation gene cluster, partial sequence." XP002380296 extrait de EBI accession no. EM_PRO:AY123973 Database accession no. AY123973
- FIORENZA STEPHANIE ET AL: "Review of MTBE biodegradation and bioremediation." BIOREMEDIATION JOURNAL, vol. 7, no. 1, mars 2003 (2003-03), pages 1-35, XP009066151 ISSN: 1088-9868
- FRANCOIS A ET AL: "Comparison of MTBE and TAME degradation pathways in Mycobacterium austroafricanum IFP 2012." FEMS CONGRESS OF EUROPEAN MICROBIOLOGISTS ABSTRACT BOOK, no. 1, 2003, page 379, XP009066153 & 1ST FEDERATION OF EUROPEAN MICROBIOLOGICAL SOCIETIES (FEMS) CONGRESS OF EUROPEAN MICROBIOLOGISTS; LJUBLJANA, SLOVENIA; JUNE 29-JULY 03, 2003
- FRANCOIS A ET AL: "Roles of tert-butyl formate, tert-butyl alcohol and acetone in the regulation of methyl tert-butyl ether degradation by Mycobacterium austroafricanum IFP 2012." APPLIED MICROBIOLOGY AND BIOTECHNOLOGY, vol. 62, no. 2-3, août 2003 (2003-08), pages 256-262, XP002379948 ISSN: 0175-7598
- PIVETEAU P ET AL: "BIODEGRADATION OF TERT-BUTYL ALCOHOL AND RELATED XENOBIOTICS BY A METHYLOTROPHIC BACTERIAL ISOLATE" APPLIED MICROBIOLOGY AND BIOTECHNOLOGY, SPRINGER VERLAG, BERLIN, DE, vol. 55, no. 3, avril 2001 (2001-04), pages 369-373, XP001036947 ISSN: 0175-7598
- DEEB RULA A ET AL: "Aerobic MTBE biodegradation: An examination of past studies, current challenges and future research directions" BIODEGRADATION, vol. 11, no. 2-3, 2000, pages 171-186, XP002379950 ISSN: 0923-9820
- HRISTOVA K R ET AL: "Characterization of enzymes involved in MTBE biodegradation in Aquabacterium sp. strain PM1." ABSTRACTS OF THE GENERAL MEETING OF THE AMERICAN SOCIETY FOR MICROBIOLOGY, vol. 103, 2003, pages Q-078 URL, XP002379951 & 103RD AMERICAN SOCIETY FOR MICROBIOLOGY GENERAL MEETING; WASHINGTON, DC, USA; MAY 18-22, 2003 ISSN: 1060-2011
- JU K ET AL: "Physiological characterization of MTBE metabolism by a new bacterial isolate." ABSTRACTS OF THE GENERAL MEETING OF THE AMERICAN SOCIETY FOR MICROBIOLOGY, vol. 103, 2003, pages Q-042 URL, XP002379952 & 103RD AMERICAN SOCIETY FOR MICROBIOLOGY GENERAL MEETING; WASHINGTON, DC, USA; MAY 18-22, 2003 ISSN: 1060-2011
- FAYOLLE F ET AL: "Microbial degradation and fate in the environment of methyl tert-butyl ether and related fuel oxygenates" APPLIED MICROBIOLOGY AND BIOTECHNOLOGY 2001 GERMANY, vol. 56, no. 3-4, 2001, pages 339-349, XP002379953 ISSN: 0175-7598
- JOHNSON E L ET AL: "Induction of Methyl Tertiary Butyl Ether (MTBE)-Oxidizing Activity in Mycobacterium vaccae JOB5 by MTBE" APPLIED AND ENVIRONMENTAL MICROBIOLOGY 2004 UNITED STATES, vol. 70, no. 2, 2004, pages 1023-1030, XP002379954 ISSN: 0099-2240
- FERREIRA NICOLAS LOPES ET AL: "Genes involved in the methyl tert-butyl ether (MTBE) metabolic pathway of Mycobacterium austroafricanum IFP 2012." MICROBIOLOGY (READING, ENGLAND) MAY 2006, vol. 152, no. Pt 5, mai 2006 (2006-05), pages 1361-1374, XP002379957 ISSN: 1350-0872
- FERREIRA N L ET AL: "Isolation and characterization of a new Mycobacterium austroafricanum strain, IFP 2015, growing on MTBE" APPLIED MICROBIOLOGY AND BIOTECHNOLOGY 2006 GERMANY, vol. 70, no. 3, 2006, pages 358-365, XP002380213 ISSN: 0175-7598

### SEQUENCE LISTING

<110> Institut Français du Pétrole National Research Council of Canada
<120> polypeptides ayant une activité dans la voie de dégradation du MTBE et leurs utilisations
<130> BCT060237
<160> 35
<170> PatentIn version 3.3
<210> 1
   <211> 1515
   <212> DNA
   <213> Mycobacterium austroafricanum
<400> 1
   atgacccgaa ctctctcggc tgatgccgac acccgcacgg cgacacctcc gctgatgtac 60
   gtcaacggcg agtggctgcc cgcccgcagt ggggccacct ttcccaccat cgaacccagc 120
   acgggtcgac cgatcactga gattccccgc ggggactcga gcgatgtgga cgcggcggtg 180
   aaagccgctg ccgacgtggc cgttgagtgg cagttcaccg atgccatcac ccgcgccgcc 240
   ctgctcaggc gattggcgga gctggtggca gagaacgccg aggagctggc gcggatcgag 300
   tcgctggact cgggtcacta tctggcgaag gcgcgtgaac tggtgaccgc gatacccctg 360
   tggctcgagt actgggccgg cgcagctgac aaagtgggcg gccgcaccat cgctgtaccg 420
   ggtaacaaac tcagcttcac cttgttggaa ccgctgggcg tcaccgcgca catcatcccg 480
   tggaactacc cgctgttgat ccttgctcgg tccatcgccc cggcactcgc attgggcaac 540
   acctgtgtcg tcaagcccgc tgaggacacg tccctgtcag cgctgaagtt cgccgagctg 600
   gtacacgccg ccggttttcc cgccggagtg ttcaatgtgg tgaccggtta cggttccgaa 660
   gccggcgcgg ctcttgccgc tcaccccgag gtgcgcggaa tcaccttcac cggttcgacc 720
   gagaccgggc gggagatcgc ccgactgggc ggccaacaca tcgcccaggt caacctggaa 780
   ctcggcggga agagcccgtt ggtcgtcttt cccgacgcgc cgctcgaaga cgccgtagag 840
   gtggccgtac agggcttctg ctcacgggca gggcaagtgt gtgtcgccgg gagccggctc 900
   ttcctccatg aggacatcgc cgaccggttc ctcgagatgc tcgtttcgcg actcgagact 960
   gtcaccgtcg gcgacccgtt cgacggtgcg acccagatgg gtccgctcgc ctcgaagaag 1020
   cactacgacc gtgtgcgtga gtacatcgag gtcgggaagc aggaggcgac cctgctctac 1080
   ggcggcggtc ggccgacgga cacgcccgat gacgggttct tcgtcgagcc aacggttttc 1140
   gtcgacgtcg caacggatgc gcggatcgca cgcgaggaga tcttcgggcc cgtcacagcg 1200
   gtgatgcggt ggtcatcggt cgacgatctg atcgccacca tcaatgattc ggaattcggt 1260
   ctcttcgctg tgctctggtg ccgggacatc accagtgcgc tggacacggc gaaacgcctg 1320
   caggtcggct cggtaatgat caacgactgg ttcggtgagc tgccgatgac tccgcacgga 1380
   ggccacaagc aaagcggcac cggacgcgag gaaggcctcg aagcggtaca cggctacaca 1440
   caggtcaagc acatcggcat caacctcgag ccgtcgcccg caaagtccgc cgattgggcc 1500
   ggtgcacctc tgtga 1515
<210> 2
   <211> 504
   <212> PRT
   <213> Mycobacterium austroafricanum
<400> 2
   Met Thr Arg Thr Leu Ser Ala Asp Ala Asp Thr Arg Thr Ala Thr Pro
   1 5 10 15
   Pro Leu Met Tyr Val Asn Gly Glu Trp Leu Pro Ala Arg Ser Gly Ala
   20 25 30
   Thr Phe Pro Thr Ile Glu Pro Ser Thr Gly Arg Pro Ile Thr Glu Ile
   35 40 45
   Pro Arg Gly Asp Ser Ser Asp Val Asp Ala Ala Val Lys Ala Ala Ala
   50 55 60
   Asp Val Ala Val Glu Trp Gln Phe Thr Asp Ala Ile Thr Arg Ala Ala
   65 70 75 80
   Leu Leu Arg Arg Leu Ala Glu Leu Val Ala Glu Asn Ala Glu Glu Leu
   85 90 95
   Ala Arg Ile Glu Ser Leu Asp Ser Gly His Tyr Leu Ala Lys Ala Arg
   100 105 110
   Glu Leu Val Thr Ala Ile Pro Leu Trp Leu Glu Tyr Trp Ala Gly Ala
   115 120 125
   Ala Asp Lys Val Gly Gly Arg Thr Ile Ala Val Pro Gly Asn Lys Leu
   130 135 140
   Ser Phe Thr Leu Leu Glu Pro Leu Gly Val Thr Ala His Ile Ile Pro
   145 150 155 160
   Trp Asn Tyr Pro Leu Leu Ile Leu Ala Arg Ser Ile Ala Pro Ala Leu
   165 170 175
   Ala Leu Gly Asn Thr Cys Val Val Lys Pro Ala Glu Asp Thr Ser Leu
   180 185 190
   Ser Ala Leu Lys Phe Ala Glu Leu Val His Ala Ala Gly Phe Pro Ala
   195 200 205
   Gly Val Phe Asn Val Val Thr Gly Tyr Gly Ser Glu Ala Gly Ala Ala
   210 215 220
   Leu Ala Ala His Pro Glu Val Arg Gly Ile Thr Phe Thr Gly Ser Thr
   225 230 235 240
   Glu Thr Gly Arg Glu Ile Ala Arg Leu Gly Gly Gln His Ile Ala Gln
   245 250 255
   Val Asn Leu Glu Leu Gly Gly Lys Ser Pro Leu Val Val Phe Pro Asp
   260 265 270
   Ala Pro Leu Glu Asp Ala Val Glu Val Ala Val Gln Gly Phe Cys Ser
   275 280 285
   Arg Ala Gly Gln Val Cys Val Ala Gly Ser Arg Leu Phe Leu His Glu
   290 295 300
   Asp Ile Ala Asp Arg Phe Leu Glu Met Leu Val Ser Arg Leu Glu Thr
   305 310 315 320
   Val Thr Val Gly Asp Pro Phe Asp Gly Ala Thr Gln Met Gly Pro Leu
   325 330 335
   Ala Ser Lys Lys His Tyr Asp Arg Val Arg Glu Tyr Ile Glu Val Gly
   340 345 350
   Lys Gln Glu Ala Thr Leu Leu Tyr Gly Gly Gly Arg Pro Thr Asp Thr
   355 360 365
   Pro Asp Asp Gly Phe Phe Val Glu Pro Thr Val Phe Val Asp Val Ala
   370 375 380
   Thr Asp Ala Arg Ile Ala Arg Glu Glu Ile Phe Gly Pro Val Thr Ala
   385 390 395 400
   Val Met Arg Trp Ser Ser Val Asp Asp Leu Ile Ala Thr Ile Asn Asp
   405 410 415
   Ser Glu Phe Gly Leu Phe Ala Val Leu Trp Cys Arg Asp Ile Thr Ser
   420 425 430
   Ala Leu Asp Thr Ala Lys Arg Leu Gln Val Gly Ser Val Met Ile Asn
   435 440 445
   Asp Trp Phe Gly Glu Leu Pro Met Thr Pro His Gly Gly His Lys Gln
   450 455 460
   Ser Gly Thr Gly Arg Glu Glu Gly Leu Glu Ala Val His Gly Tyr Thr
   465 470 475 480
   Gln Val Lys His Ile Gly Ile Asn Leu Glu Pro Ser Pro Ala Lys Ser
   485 490 495
   Ala Asp Trp Ala Gly Ala Pro Leu
   500
<210> 3
   <211> 648
   <212> DNA
   <213> Mycobacterium austroafricanum
<400> 3
   atgcctcaag aagacgcctt ggtgagcgcc ccattcgggg cgccgtcctt gggggaagat    60
   cgtcccgatc aaccccattt cgagaccggc cgaccgagac tcggcggact ggtcgccccc   120
   gtcgcgtttg atgagctcga ggcagcggtc atcgacgccc tggccgacac gatgatcccc   180
   gccgaaggcg gctttccggc cgcaagcgac gtgggaatcg tcgatttctt cggccgctac   240
   acgactccca ccggattccg cgcgaagcac ttcccctacc tcgaagagga caagttgaag   300
   agcgcactcg cggggctcgg cgaagaattc gtcaacgccg acaccgatac gcgcacccag   360
   gcggtcctcc gattggagaa ggacgatgag gagttcttcg cgcaggtgag aagcctcgtg   420
   tattacggct actactccgc gaacgcagtg accgtcgcca ttcaccagca gattccggcc   480
   gggcgcgact accacggacc cccactcccc tacggctatc tgcattgcat cgaggactgg   540
   gatgaagcgg cgctctccac atcggggcag ggctcaggct atgtcgccac cgacgatgtg   600
   gtccgagtgg atctcagcaa actcacctgg ctgaacaaca agacttga                648
<210> 4
   <211> 215
   <212> PRT
   <213> Mycobacterium austroafricanum
<400> 4
   Met Pro Gln Glu Asp Ala Leu Val Ser Ala Pro Phe Gly Ala Pro Ser
   1               5                   10                  15
   Leu Gly Glu Asp Arg Pro Asp Gln Pro His Phe Glu Thr Gly Arg Pro
               20                  25                  30
   Arg Leu Gly Gly Leu Val Ala Pro Val Ala Phe Asp Glu Leu Glu Ala
           35                  40                  45
   Ala Val Ile Asp Ala Leu Ala Asp Thr Met Ile Pro Ala Glu Gly Gly
       50                  55                  60
   Phe Pro Ala Ala Ser Asp Val Gly Ile Val Asp Phe Phe Gly Arg Tyr
   65                  70                  75                  80
   Thr Thr Pro Thr Gly Phe Arg Ala Lys His Phe Pro Tyr Leu Glu Glu
                   85                  90                  95
   Asp Lys Leu Lys Ser Ala Leu Ala Gly Leu Gly Glu Glu Phe Val Asn
               100                 105                 110
   Ala Asp Thr Asp Thr Arg Thr Gln Ala Val Leu Arg Leu Glu Lys Asp
           115                 120                 125
   Asp Glu Glu Phe Phe Ala Gln Val Arg Ser Leu Val Tyr Tyr Gly Tyr
       130                 135                 140
   Tyr Ser Ala Asn Ala Val Thr Val Ala Ile His Gln Gln Ile Pro Ala
   145                 150                 155                 160
   Gly Arg Asp Tyr His Gly Pro Pro Leu Pro Tyr Gly Tyr Leu His Cys
                   165                 170                 175
   Ile Glu Asp Trp Asp Glu Ala Ala Leu Ser Thr Ser Gly Gln Gly Ser
               180                 185                 190
   Gly Tyr Val Ala Thr Asp Asp Val Val Arg Val Asp Leu Ser Lys Leu
           195                 200                 205
   Thr Trp Leu Asn Asn Lys Thr
       210                 215
<210> 5
   <211> 1659
   <212> DNA
   <213> Mycobacterium austroafricanum
<400> 5
   gtgacaacat ccgccgatca gaccgacgtt ctggtcatcg gttcgggacc gggcggcgcg 60
   ggcgtcacgc tcaagctggt gcaggccgga tacaaggtga cctgcctgga gcaggggcct 120
   tgggtgacac cgcccgagca cccgcattac caccgggaat gggagatcga aaagcaacgc 180
   ggatgggcct acgacccgaa cgtccgtgga ctcccggaag actacccggt gaccggcttc 240
   accacgcctt atctcatgaa caacgtgggc ggtagcacga tgcactacgc cggccactgg 300
   ccgcgctaca agccggtcga cttccgcaag ggcaccgagc acgggttgga aggcacgatc 360
   gactggccga tcagctacga agagctcgcg ccgtactacg acgagaacga cgcgatctac 420
   ggcatctccg gcatggtggg cgatccgtcg tatccggatc gaaccggagt cgaccgcgat 480
   ccaccggtca aaccgggcaa gctggggcgc aacttcgctc aggcgctggg cgacctgggt 540
   tggcactggt ggccatcgga caacgcgatc atcactcggc cacgcgaagg ccgcgaggct 600
   gacatcgccg caggcaacga gctctcgggt agcccgacgg gatcgctcag cacgccgacg 660
   cacacccact ggccgaccgc catcgcgctc ggagcggact tgcgtaccca cgcccgagtc 720
   gaacagatcc acacgaagaa cggcaaggcc accggtgcga cctacatcga cacccgtacc 780
   ggcgcacggc acgagatcaa cgcgaagatc gtggtggtct cggccagcgg gatcggaacc 840
   ccgagactgc tcctcatgag cgcgcagaag gggcatcccg acggtctggc caacagcaac 900
   ggtctcgtcg gcaaatacct gatgcaccac attcttcgcg ttctggcgag cgtggttcgc 960
   acaagccgga tggaaggcta caagggagcc ttcggcgctc cactgtattc gcacgagttc 1020
   taccacaccg acaccaatcg cggcttcgtc aacggtttcg gcatgcaggt ggcacgcagc 1080
   ttcggcgctg catacacagc aatgggcagc cacaccggtt acgtggcccc ctggggcaaa 1140
   tcgcatcgca agttcttcaa cgaacacttc ggcaatcact tgatggtttt catgttcggc 1200
   gaagacctcc ccgtcgagac gaactgcgtg acactcgatc ctgacgccaa agactcgagt 1260
   ggcctccctg cggcgcgtgt caactgggaa ccacacgaga acgacatcgc gctggccaat 1320
   tacggcatcg accggatctt cgaggccgcg cgagcgttgg gcgctgtcga gaccaacgac 1380
   accggcgtgc tcaatcctcc gcccggttgg cacttgatgg gcacctgtcg gatgggtaac 1440
   aacccagaag attcggtcac caacaagtgg catcagacct gggatgtgcc gaatctcttc 1500
   gttgtcgacg ggagctcgct caccaccggc ggagctgtca acccgacatc gacgatcggc 1560
   gcgctcgccg tgcgggcagg agattacatc tcccgccgat tctccgacat cgtcgatcag 1620
   cgcaccacgc cgagcaacga agacgcacct gccatctaa 1659
<210> 6
   <211> 552
   <212> PRT
   <213> Mycobacterium austroafricanum
<400> 6
   Met Thr Thr Ser Ala Asp Gln Thr Asp Val Leu Val Ile Gly Ser Gly
   1 5 10 15
   Pro Gly Gly Ala Gly Val Thr Leu Lys Leu Val Gln Ala Gly Tyr Lys
   20 25 30
   Val Thr Cys Leu Glu Gln Gly Pro Trp Val Thr Pro Pro Glu His Pro
   35 40 45
   His Tyr His Arg Glu Trp Glu Ile Glu Lys Gln Arg Gly Trp Ala Tyr
   50 55 60
   Asp Pro Asn Val Arg Gly Leu Pro Glu Asp Tyr Pro Val Thr Gly Phe
   65 70 75 80
   Thr Thr Pro Tyr Leu Met Asn Asn Val Gly Gly Ser Thr Met His Tyr
   85 90 95
   Ala Gly His Trp Pro Arg Tyr Lys Pro Val Asp Phe Arg Lys Gly Thr
   100 105 110
   Glu His Gly Leu Glu Gly Thr Ile Asp Trp Pro Ile Ser Tyr Glu Glu
   115 120 125
   Leu Ala Pro Tyr Tyr Asp Glu Asn Asp Ala Ile Tyr Gly Ile Ser Gly
   130 135 140
   Met Val Gly Asp Pro Ser Tyr Pro Asp Arg Thr Gly Val Asp Arg Asp
   145 150 155 160
   Pro Pro Val Lys Pro Gly Lys Leu Gly Arg Asn Phe Ala Gln Ala Leu
   165 170 175
   Gly Asp Leu Gly Trp His Trp Trp Pro Ser Asp Asn Ala Ile Ile Thr
   180 185 190
   Arg Pro Arg Glu Gly Arg Glu Ala Asp Ile Ala Ala Gly Asn Glu Leu
   195 200 205
   Ser Gly Ser Pro Thr Gly Ser Leu Ser Thr Pro Thr His Thr His Trp
   210 215 220
   Pro Thr Ala Ile Ala Leu Gly Ala Asp Leu Arg Thr His Ala Arg Val
   225 230 235 240
   Glu Gln Ile His Thr Lys Asn Gly Lys Ala Thr Gly Ala Thr Tyr Ile
   245 250 255
   Asp Thr Arg Thr Gly Ala Arg His Glu Ile Asn Ala Lys Ile Val Val
   260 265 270
   Val Ser Ala Ser Gly Ile Gly Thr Pro Arg Leu Leu Leu Met Ser Ala
   275 280 285
   Gln Lys Gly His Pro Asp Gly Leu Ala Asn Ser Asn Gly Leu Val Gly
   290 295 300
   Lys Tyr Leu Met His His Ile Leu Arg Val Leu Ala Ser Val Val Arg
   305 310 315 320
   Thr Ser Arg Met Glu Gly Tyr Lys Gly Ala Phe Gly Ala Pro Leu Tyr
   325 330 335
   Ser His Glu Phe Tyr His Thr Asp Thr Asn Arg Gly Phe Val Asn Gly
   340 345 350
   Phe Gly Met Gln Val Ala Arg Ser Phe Gly Ala Ala Tyr Thr Ala Met
   355 360 365
   Gly Ser His Thr Gly Tyr Val Ala Pro Trp Gly Lys Ser His Arg Lys
   370 375 380
   Phe Phe Asn Glu His Phe Gly Asn His Leu Met Val Phe Met Phe Gly
   385 390 395 400
   Glu Asp Leu Pro Val Glu Thr Asn Cys Val Thr Leu Asp Pro Asp Ala
   405 410 415
   Lys Asp Ser Ser Gly Leu Pro Ala Ala Arg Val Asn Trp Glu Pro His
   420 425 430
   Glu Asn Asp Ile Ala Leu Ala Asn Tyr Gly Ile Asp Arg Ile Phe Glu
   435 440 445
   Ala Ala Arg Ala Leu Gly Ala Val Glu Thr Asn Asp Thr Gly Val Leu
   450 455 460
   Asn Pro Pro Pro Gly Trp His Leu Met Gly Thr Cys Arg Met Gly Asn
   465 470 475 480
   Asn Pro Glu Asp Ser Val Thr Asn Lys Trp His Gln Thr Trp Asp Val
   485 490 495
   Pro Asn Leu Phe Val Val Asp Gly Ser Ser Leu Thr Thr Gly Gly Ala
   500 505 510
   Val Asn Pro Thr Ser Thr Ile Gly Ala Leu Ala Val Arg Ala Gly Asp
   515 520 525
   Tyr Ile Ser Arg Arg Phe Ser Asp Ile Val Asp Gln Arg Thr Thr Pro
   530 535 540
   Ser Asn Glu Asp Ala Pro Ala Ile
   545 550
<210> 7
   <211> 672
   <212> DNA
   <213> Mycobacterium austroafricanum
<400> 7
   atgtcggcaa ccgacgtccc gccgcttccc caatccatcg gcccactgca cccggacgtt 60
   gaagccggca gcttgtccga accaggtctc gagaccgcgg cgggccgctg ggtagctttc 120
   ggcctggcca atctggcggt ggtgctggca gtgtccttgg cggggtggta cctgctcgcc 180
   gacccccgac tcagcccgtg gtccttctat ccactcccgt tcaacgcggc tctgttctgg 240
   gccatcctct tcgttgtgtt catcggcttc aatgccggat tcgcagggtt catccgactt 300
   tcacagccat ggcggggact cgccatcacc gtcgccacgg gcatcttcgc ggtcgccgtg 360
   acgtgggtgc tggcagccgg actcggcagt gtgaacgctg atttcgctgc cggccgagac 420
   ggcggcctcg gctacttcac cggcgcgctg ttcgtgctct tcgggttcgg taccttcgtg 480
   atagtcgtcc tcaattggca gcactggcca tggccccaac tcggtctctc gcagcccggc 540
   gtgggattgg ccgagatcgc cgcggttgcc ggcccgacca tgctgctgta cttcgccttc 600
   ggcttgcccg cagtcagcgc aggcggtgcc gaacccgtgt tggaattgga caccctcatg 660
   ggctggttct ag 672
<210> 8
   <211> 223
   <212> PRT
   <213> Mycobacterium austroafricanum
<400> 8
   Met Ser Ala Thr Asp Val Pro Pro Leu Pro Gln Ser Ile Gly Pro Leu
   1               5                   10                  15
   His Pro Asp Val Glu Ala Gly Ser Leu Ser Glu Pro Gly Leu Glu Thr
               20                  25                  30
   Ala Ala Gly Arg Trp Val Ala Phe Gly Leu Ala Asn Leu Ala Val Val
           35                  40                  45
   Leu Ala Val Ser Leu Ala Gly Trp Tyr Leu Leu Ala Asp Pro Arg Leu
       50                  55                  60
   Ser Pro Trp Ser Phe Tyr Pro Leu Pro Phe Asn Ala Ala Leu Phe Trp
   65                  70                  75                  80
   Ala Ile Leu Phe Val Val Phe Ile Gly Phe Asn Ala Gly Phe Ala Gly
                   85                  90                  95
   Phe Ile Arg Leu Ser Gln Pro Trp Arg Gly Leu Ala Ile Thr Val Ala
               100                 105                 110
   Thr Gly Ile Phe Ala Val Ala Val Thr Trp Val Leu Ala Ala Gly Leu
           115                 120                 125
   Gly Ser Val Asn Ala Asp Phe Ala Ala Gly Arg Asp Gly Gly Leu Gly
       130                 135                 140
   Tyr Phe Thr Gly Ala Leu Phe Val Leu Phe Gly Phe Gly Thr Phe Val
   145                 150                 155                 160
   Ile Val Val Leu Asn Trp Gln His Trp Pro Trp Pro Gln Leu Gly Leu
                   165                 170                 175
   Ser Gln Pro Gly Val Gly Leu Ala Glu Ile Ala Ala Val Ala Gly Pro
               180                 185                 190
   Thr Met Leu Leu Tyr Phe Ala Phe Gly Leu Pro Ala Val Ser Ala Gly
           195                 200                 205
   Gly Ala Glu Pro Val Leu Glu Leu Asp Thr Leu Met Gly Trp Phe
       210                 215                 220
<210> 9
   <211> 1233
   <212> DNA
   <213> Mycobacterium austroafricanum
<400> 9
   tcagtcttgt tgacctatag cgctcagtgt tgtgcgcagt gcccgcactg cgggcgctac 60
   cgatggcgcc aacaaacctg ccatgttcgc tggatagcgg acggtcacga cggcgggttc 120
   ggcgaaccga tagggtcgac ccaccacgat gcccgcgaat tgctttctga gtcgggacat 180
   ctccactcga acggctccaa cgcgtgtggc gtctccgtac aggtctgccg caagcgcagg 240
   cgcagagcgg ccgtcagcgc tgtgggccag tatgagcagt atctcggcgt gccgcagtga 300
   aatgtcgtgc cgccagttac cgaatcggcc ctccatctcg agcgcaggag aggcactgtc 360
   gtgcagattc aacgtgaccc gtgtgactgc tgactccgtg tcgcttccca ccagtcgcac 420
   caaccacccg ccgggtagca tctcgacctc gcaatcgccg agtgacggca cccataagtg 480
   gccgggcgcg atatcggcgg gcagcatgat ccgcctctgc acggtcaacg agtccactgc 540
   ggccacccaa ccgtccccat ccacggccac ggcaggcgac ccgactcgcg ccagtatcgg 600
   agcagcgacc gagcgcagcc ggttcagtgc gagttcgtgc tgcccacgca actccgattc 660
   ggccaaccgt gccacggcgt cgatgagggc gaccgtcacc ggatgcacgg tcgccgccgg 720
   tccggtcacg tcgaccacac cgatcacgcg gccggtgcgc ggactacgga tcggtgcgcc 780
   cgcgcacgtc cacgggtgtt ggctgcggtt gtaatgctcc gcactgaagg tctggacggc 840
   gcgctgtgag acgagagcgg taccgagggc gttcgtaccg accgtcgttt cggcccactg 900
   ggctccttcg acgaacccaa gcctgtccgc gttcgccagc acacgaggcg agccggatcg 960
   ccacagcaca gcgcactcgg catcggcgac ggcaacgata ctgtcgcccg tggacgtcaa 1020
   cggggcgagg ccgcgcgtca actccccgac caccgaagcc aagccggatt cggacctcaa 1080
   cacctcgacc gtgtccgact ccatcacggg cggcggtcga cggtcgggtc gcaaacccct 1140
   ggccatgagt cgctgccagg aatcccagat gacacgacgt ggacgggccg gagcttgccc 1200
   gcccgccatc gtggcctcgt agacagccgc cat 1233
<210> 10
   <211> 410
   <212> PRT
   <213> Mycobacterium austroafricanum
<400> 10
   Met Ala Ala Val Tyr Glu Ala Thr Met Ala Gly Gly Gln Ala Pro Ala
   1               5                   10                  15
   Arg Pro Arg Arg Val Ile Trp Asp Ser Trp Gln Arg Leu Met Ala Arg
               20                  25                  30
   Gly Leu Arg Pro Asp Arg Arg Pro Pro Pro Val Met Glu Ser Asp Thr
           35                  40                  45
   Val Glu Val Leu Arg Ser Glu Ser Gly Leu Ala Ser Val Val Gly Glu
       50                  55                  60
   Leu Thr Arg Gly Leu Ala Pro Leu Thr Ser Thr Gly Asp Ser Ile Val
   65                  70                  75                  80
   Ala Val Ala Asp Ala Glu Cys Ala Val Leu Trp Arg Ser Gly Ser Pro
   85 90 95
   Arg Val Leu Ala Asn Ala Asp Arg Leu Gly Phe Val Glu Gly Ala Gln
   100 105 110
   Trp Ala Glu Thr Thr Val Gly Thr Asn Ala Leu Gly Thr Ala Leu Val
   115 120 125
   Ser Gln Arg Ala Val Gln Thr Phe Ser Ala Glu His Tyr Asn Arg Ser
   130 135 140
   Gln His Pro Trp Thr Cys Ala Gly Ala Pro Ile Arg Ser Pro Arg Thr
   145 150 155 160
   Gly Arg Val Ile Gly Val Val Asp Val Thr Gly Pro Ala Ala Thr Val
   165 170 175
   His Pro Val Thr Val Ala Leu Ile Asp Ala Val Ala Arg Leu Ala Glu
   180 185 190
   Ser Glu Leu Arg Gly Gln His Glu Leu Ala Leu Asn Arg Leu Arg Ser
   195 200 205
   Val Ala Ala Pro Ile Leu Ala Arg Val Gly Ser Pro Ala Val Ala Val
   210 215 220
   Asp Gly Asp Gly Trp Val Ala Ala Val Asp Ser Leu Thr Val Gln Arg
   225 230 235 240
   Arg Ile Met Leu Pro Ala Asp Ile Ala Pro Gly His Leu Trp Val Pro
   245 250 255
   Ser Leu Gly Asp Cys Glu Val Glu Met Leu Pro Gly Gly Trp Leu Val
   260 265 270
   Arg Leu Val Gly Ser Asp Thr Glu Ser Ala Val Thr Arg Val Thr Leu
   275 280 285
   Asn Leu His Asp Ser Ala Ser Pro Ala Leu Glu Met Glu Gly Arg Phe
   290 295 300
   Gly Asn Trp Arg His Asp Ile Ser Leu Arg His Ala Glu Ile Leu Leu
   305 310 315 320
   Ile Leu Ala His Ser Ala Asp Gly Arg Ser Ala Pro Ala Leu Ala Ala
   325 330 335
   Asp Leu Tyr Gly Asp Ala Thr Arg Val Gly Ala Val Arg Val Glu Met
   340 345 350
   Ser Arg Leu Arg Lys Gln Phe Ala Gly Ile Val Val Gly Arg Pro Tyr
   355 360 365
   Arg Phe Ala Glu Pro Ala Val Val Thr Val Arg Tyr Pro Ala Asn Met
   370 375 380
   Ala Gly Leu Leu Ala Pro Ser Val Ala Pro Ala Val Arg Ala Leu Arg
   385 390 395 400
   Thr Thr Leu Ser Ala Ile Gly Gln Gln Asp
   405 410
<210> 11
   <211> 10327
   <212> DNA
   <213> Mycobacterium austroafricanum
<400> 11
   tgcagcacag cacccgcacc ccaccggcgg cgatacctcg ccacccggct cacccgcgcg     60
   gtaggcaccc accgcaacaa cacgggcaga gcggtccagc acctgtggag aatcatcgac    120
   accctcaccg actaccttcg cgccgtcgac agcggtcacc gcacctacac cgaagcgttc    180
   cacgaactgc tgccctacta cgccgacgca tcacaagccg ttgggactgc gaccaatccg    240
   gcctgacgac atgaccaacc cgggacatat gggcgccgaa tcgggcgttt cgccgacagc    300
   ccacggcacc ctcaaggcgt ccttctgacc caaccgaagt gcagcctatt cacctgaatg    360
   acagcggcgc ctattgttgg acacatggtg tcggctacac ctcacgagtc ccgctcggtc    420
   actgcgcaac gctcgtcggg tgacgaacag cgcctcgacg ccgcgtatgc ggaactggcc    480
   acgtcgttca acgaagaagt caccgacgcc gagagacgcg cggcgcgcac caagtacatc    540
   gaccgccgcg aacccggccg tcaatgaatg cggcgcgcat cttgtcgtcg gccttctcat    600
   atcaagtatc gaatggtcac acagggcgcg gcgaccggtg gggccgtccc atgggcactt    660
   cccggcttcc ttcgaccact cgccacgggg ccggcaggac gtcgcctcga cgcgccaact    720
   caaatgacgc tgccaatcac aagcccatct caccgacgct ggcggtccta tgcatcgggt    780
   caccaggtcg actgagaacg gacaaagtgg gtccggattg gtcgacgaac tgcgcgaggc    840
   cggctgcgcc gtgggtgtag tgaagggccg cacccacggc gctgatcaag acagaccgta    900
   gcggcgtcgc agctcctcgc cgctgatcgt gttccccgcc gcataatccg catccgcggc    960
   ggccagttca tcgactaacc gctcattggc agcatggatt gcatcagcca atgctgcgaa   1020
   gtcggccggc gtgctgccag caggatcgtc actcatggct ccattctcgt cacaaccgac   1080
   tcgcccgtgg cgcgacggat ggacccgagc cgcggcgatc cgctccaact cttttagcga   1140
   cagcacgtag tccgctagtt agtattggcg gtcgcgctgg cctcgatgcc gcggtgttcg   1200
   ccgagcatcc gcttgtaggt gtgttacggt ttggcgattt gtctcactgt ttttgtgatc   1260
   tctgtttcga cctgagccgc ctcttgaatt tgttcggtaa gcgatcggtc ggccaactgg   1320
   gtgtttctgt tcgcatgtct gcatattgtt cctattcatg caccactgat tactcttgtg   1380
   gagaataagc gtgtcggtcc caacatgtat tatctgaacg gtgagtagag aaagagcggc 1440
   gcttaagcct tgctgactac agcgtgccaa agacgcctct agtacgcgcg attgcttaac 1500
   cggaaccgta cccagccgcg cacccgcacg gcagtccgcc acggcggctc cctgaacaat 1560
   caccccaccg aggctgaaaa gctccgggag tcgataaaca gatttgtcag tcagtccaag 1620
   ctattcgaaa gcaacgtcgt gcctagtctc gctgatactt tggcgaagat ccaacagaca 1680
   acgatgcaaa gcttgcgccc gatctacgtc ccgcagctgc ccgaatcgat gttcgacagc 1740
   cttcgcaaga ccttaaaggg catctttccc gctaattggc cgagcgaatt gcctgacttc 1800
   gaccgcatcg aggaggtcat agagaaggac ggcatcccta tcgtccacat cccgcgagct 1860
   gagatcgtcc aagagatctg cgacgctcac gactacgacg cacgcgtgca aatcttggtc 1920
   gaccgtgcaa gcgacatcgc tgacgattgc cgagatgcac ttcagcgtgt ctacgacgag 1980
   atagtggata agcagctccc cctagcccag cgagcgatcg aggcatacca ggctggatac 2040
   ttcgagtctg cccaggcatt ggcggtgagt gtgtgcgata cctatctgaa gaagatgtat 2100
   caaggcacgc gctatgcgga catacaaagt gatctcacgc tcgataactc gaacgaggtg 2160
   gccgtcgccg tcgccttcaa ctttcacttc gcattggcat ccgcagttcc gttcctcacc 2220
   ccctggaacc ctgcgagggg tgaaaagccg cccacgaggc tgtcacgcac gcgagtatcc 2280
   atgacgcaag caccgaccat atgacgctgc tgaatgcgac gattgcgata atgctcctga 2340
   ccagcatgac tgttgctatc gactacatgg ctcggcgcgt gcggaagaac aagggagatt 2400
   gcgacacctg aagtcctcgc acgccgcgtg acaggtacgc ctcctctatc cacgtcggcg 2460
   aaggcgatcg cttaaccgcg attgcggact tggcaacatc atcaagacgc cctccttcac 2520
   ctccgtctca atgcagttgg catcctcaca ggtcgcgagt gacacctcta ctgagaccgg 2580
   acaactccgc gcgccgcggc caaccacacc accatcaccg acatcgaagc cgccctgcgc 2640
   gaccaacggc tcctgaaccc cgcgcccgca accatcgccg acgccgcacc ctatggcgcc 2700
   agctcggtga ctggccacct cacgcgtaca tcacgccggc gactcccgca tcggacgctt 2760
   aagtcagtct tgttgaccta tagcgctcag tgttgtgcgc agtgcccgca ctgcgggcgc 2820
   taccgatggc gccaacaaac ctgccatgtt cgctggatag cggacggtca cgacggcggg 2880
   ttcggcgaac cgatagggtc gacccaccac gatgcccgcg aattgctttc tgagtcggga 2940
   catctccact cgaacggctc caacgcgtgt ggcgtctccg tacaggtctg ccgcaagcgc 3000
   aggcgcagag cggccgtcag cgctgtgggc cagtatgagc agtatctcgg cgtgccgcag 3060
   tgaaatgtcg tgccgccagt taccgaatcg gccctccatc tcgagcgcag gagaggcact 3120
   gtcgtgcaga ttcaacgtga cccgtgtgac tgctgactcc gtgtcgcttc ccaccagtcg 3180
   caccaaccac ccgccgggta gcatctcgac ctcgcaatcg ccgagtgacg gcacccataa 3240
   gtggccgggc gcgatatcgg cgggcagcat gatccgcctc tgcacggtca acgagtccac 3300
   tgcggccacc caaccgtccc catccacggc cacggcaggc gacccgactc gcgccagtat 3360
   cggagcagcg accgagcgca gccggttcag tgcgagttcg tgctgcccac gcaactccga 3420
   ttcggccaac cgtgccacgg cgtcgatgag ggcgaccgtc accggatgca cggtcgccgc 3480
   cggtccggtc acgtcgacca caccgatcac gcggccggtg cgcggactac ggatcggtgc 3540
   gcccgcgcac gtccacgggt gttggctgcg gttgtaatgc tccgcactga aggtctggac 3600
   ggcgcgctgt gagacgagag cggtaccgag ggcgttcgta ccgaccgtcg tttcggccca 3660
   ctgggctcct tcgacgaacc caagcctgtc cgcgttcgcc agcacacgag gcgagccgga 3720
   tcgccacagc acagcgcact cggcatcggc gacggcaacg atactgtcgc ccgtggacgt 3780
   caacggggcg aggccgcgcg tcaactcccc gaccaccgaa gccaagccgg attcggacct 3840
   caacacctcg accgtgtccg actccatcac gggcggcggt cgacggtcgg gtcgcaaacc 3900
   cctggccatg agtcgctgcc aggaatccca gatgacacga cgtggacggg ccggagcttg 3960
   cccgcccgcc atcgtggcct cgtagacagc cgccatcagc cgcgagtagt cgcgcggatg 4020
   cctgtccggg cgcgatcgcg ggctccaggt cgggcagtgc tgtgcatcac ctccatcatg 4080
   ccctaccgtg ctgcggtcct accacaggaa ccgccaggtg acgcctcgaa ttcaggagta 4140
   cgcccggcgc tgtaacgccc gtgtaactct tgcgggcgca cccctcgtga atgtgtgctg 4200
   cttcacacga acggccgtac atgccgctcg tcacagcggt cggaacagac agtgacctgc 4260
   ctgtccggcg gccgggcccg cctcggtgaa tcaccgaccc ggcacgcgag tcattcatag 4320
   gagcgagaac aacatgaccc gaactctctc ggctgatgcc gacacccgca cggcgacacc 4380
   tccgctgatg tacgtcaacg gcgagtggct gcccgcccgc agtggggcca cctttcccac 4440
   catcgaaccc agcacgggtc gaccgatcac tgagattccc cgcggggact cgagcgatgt 4500
   ggacgcggcg gtgaaagccg ctgccgacgt ggccgttgag tggcagttca ccgatgccat 4560
   cacccgcgcc gccctgctca ggcgattggc ggagctggtg gcagagaacg ccgaggagct 4620
   ggcgcggatc gagtcgctgg actcgggtca ctatctggcg aaggcgcgtg aactggtgac 4680
   cgcgataccc ctgtggctcg agtactgggc cggcgcagct gacaaagtgg gcggccgcac 4740
   catcgctgta ccgggtaaca aactcagctt caccttgttg gaaccgctgg gcgtcaccgc 4800
   gcacatcatc ccgtggaact acccgctgtt gatccttgct cggtccatcg ccccggcact 4860
   cgcattgggc aacacctgtg tcgtcaagcc cgctgaggac acgtccctgt cagcgctgaa 4920
   gttcgccgag ctggtacacg ccgccggttt tcccgccgga gtgttcaatg tggtgaccgg 4980
   ttacggttcc gaagccggcg cggctcttgc cgctcacccc gaggtgcgcg gaatcacctt 5040
   caccggttcg accgagaccg ggcgggagat cgcccgactg ggcggccaac acatcgccca 5100
   ggtcaacctg gaactcggcg ggaagagccc gttggtcgtc tttcccgacg cgccgctcga 5160
   agacgccgta gaggtggccg tacagggctt ctgctcacgg gcagggcaag tgtgtgtcgc 5220
   cgggagccgg ctcttcctcc atgaggacat cgccgaccgg ttcctcgaga tgctcgtttc 5280
   gcgactcgag actgtcaccg tcggcgaccc gttcgacggt gcgacccaga tgggtccgct 5340
   cgcctcgaag aagcactacg accgtgtgcg tgagtacatc gaggtcggga agcaggaggc 5400
   gaccctgctc tacggcggcg gtcggccgac ggacacgccc gatgacgggt tcttcgtcga 5460
   gccaacggtt ttcgtcgacg tcgcaacgga tgcgcggatc gcacgcgagg agatcttcgg 5520
   gcccgtcaca gcggtgatgc ggtggtcatc ggtcgacgat ctgatcgcca ccatcaatga 5580
   ttcggaattc ggtctcttcg ctgtgctctg gtgccgggac atcaccagtg cgctggacac 5640
   ggcgaaacgc ctgcaggtcg gctcggtaat gatcaacgac tggttcggtg agctgccgat 5700
   gactccgcac ggaggccaca agcaaagcgg caccggacgc gaggaaggcc tcgaagcggt 5760
   acacggctac acacaggtca agcacatcgg catcaacctc gagccgtcgc ccgcaaagtc 5820
   cgccgattgg gccggtgcac ctctgtgatc gatcggccgc ttgccgtgcg gtgctgaaca 5880
   acgcattcca agaacaacac acatgaagga gagtcaatgc ctcaagaaga cgccttggtg 5940
   agcgccccat tcggggcgcc gtccttgggg gaagatcgtc ccgatcaacc ccatttcgag 6000
   accggccgac cgagactcgg cggactggtc gcccccgtcg cgtttgatga gctcgaggca 6060
   gcggtcatcg acgccctggc cgacacgatg atccccgccg aaggcggctt tccggccgca 6120
   agcgacgtgg gaatcgtcga tttcttcggc cgctacacga ctcccaccgg attccgcgcg 6180
   aagcacttcc cctacctcga agaggacaag ttgaagagcg cactcgcggg gctcggcgaa 6240
   gaattcgtca acgccgacac cgatacgcgc acccaggcgg tcctccgatt ggagaaggac 6300
   gatgaggagt tcttcgcgca ggtgagaagc ctcgtgtatt acggctacta ctccgcgaac 6360
   gcagtgaccg tcgccattca ccagcagatt ccggccgggc gcgactacca cggaccccca 6420
   ctcccctacg gctatctgca ttgcatcgag gactgggatg aagcggcgct ctccacatcg 6480
   gggcagggct caggctatgt cgccaccgac gatgtggtcc gagtggatct cagcaaactc 6540
   acctggctga acaacaagac ttgaacacaa gacttgacga ggagcacaag tgacaacatc 6600
   cgccgatcag accgacgttc tggtcatcgg ttcgggaccg ggcggcgcgg gcgtcacgct 6660
   caagctggtg caggccggat acaaggtgac ctgcctggag caggggcctt gggtgacacc 6720
   gcccgagcac ccgcattacc accgggaatg ggagatcgaa aagcaacgcg gatgggccta 6780
   cgacccgaac gtccgtggac tcccggaaga ctacccggtg accggcttca ccacgcctta 6840
   tctcatgaac aacgtgggcg gtagcacgat gcactacgcc ggccactggc cgcgctacaa 6900
   gccggtcgac ttccgcaagg gcaccgagca cgggttggaa ggcacgatcg actggccgat 6960
   cagctacgaa gagctcgcgc cgtactacga cgagaacgac gcgatctacg gcatctccgg 7020
   catggtgggc gatccgtcgt atccggatcg aaccggagtc gaccgcgatc caccggtcaa 7080
   accgggcaag ctggggcgca acttcgctca ggcgctgggc gacctgggtt ggcactggtg 7140
   gccatcggac aacgcgatca tcactcggcc acgcgaaggc cgcgaggctg acatcgccgc 7200
   aggcaacgag ctctcgggta gcccgacggg atcgctcagc acgccgacgc acacccactg 7260
   gccgaccgcc atcgcgctcg gagcggactt gcgtacccac gcccgagtcg aacagatcca 7320
   cacgaagaac ggcaaggcca ccggtgcgac ctacatcgac acccgtaccg gcgcacggca 7380
   cgagatcaac gcgaagatcg tggtggtctc ggccagcggg atcggaaccc cgagactgct 7440
   cctcatgagc gcgcagaagg ggcatcccga cggtctggcc aacagcaacg gtctcgtcgg 7500
   caaatacctg atgcaccaca ttcttcgcgt tctggcgagc gtggttcgca caagccggat 7560
   ggaaggctac aagggagcct tcggcgctcc actgtattcg cacgagttct accacaccga 7620
   caccaatcgc ggcttcgtca acggtttcgg catgcaggtg gcacgcagct tcggcgctgc 7680
   atacacagca atgggcagcc acaccggtta cgtggccccc tggggcaaat cgcatcgcaa 7740
   gttcttcaac gaacacttcg gcaatcactt gatggttttc atgttcggcg aagacctccc 7800
   cgtcgagacg aactgcgtga cactcgatcc tgacgccaaa gactcgagtg gcctccctgc 7860
   ggcgcgtgtc aactgggaac cacacgagaa cgacatcgcg ctggccaatt acggcatcga 7920
   ccggatcttc gaggccgcgc gagcgttggg cgctgtcgag accaacgaca ccggcgtgct 7980
   caatcctccg cccggttggc acttgatggg cacctgtcgg atgggtaaca acccagaaga 8040
   ttcggtcacc aacaagtggc atcagacctg ggatgtgccg aatctcttcg ttgtcgacgg 8100
   gagctcgctc accaccggcg gagctgtcaa cccgacatcg acgatcggcg cgctcgccgt 8160
   gcgggcagga gattacatct cccgccgatt ctccgacatc gtcgatcagc gcaccacgcc 8220
   gagcaacgaa gacgcacctg ccatctaatc cattcgaact gccgatccac ctcccgcggg 8280
   cggcgggagg tggatcggtg tccatttgat ctcttcgagc ccacaggagc accccagatg 8340
   tcggcaaccg acgtcccgcc gcttccccaa tccatcggcc cactgcaccc ggacgttgaa 8400
   gccggcagct tgtccgaacc aggtctcgag accgcggcgg gccgctgggt agctttcggc 8460
   ctggccaatc tggcggtggt gctggcagtg tccttggcgg ggtggtacct gctcgccgac 8520
   ccccgactca gcccgtggtc cttctatcca ctcccgttca acgcggctct gttctgggcc 8580
   atcctcttcg ttgtgttcat cggcttcaat gccggattcg cagggttcat ccgactttca 8640
   cagccatggc ggggactcgc catcaccgtc gccacgggca tcttcgcggt cgccgtgacg 8700
   tgggtgctgg cagccggact cggcagtgtg aacgctgatt tcgctgccgg ccgagacggc 8760
   ggcctcggct acttcaccgg cgcgctgttc gtgctcttcg ggttcggtac cttcgtgata 8820
   gtcgtcctca attggcagca ctggccatgg ccccaactcg gtctctcgca gcccggcgtg 8880
   ggattggccg agatcgccgc ggttgccggc ccgaccatgc tgctgtactt cgccttcggc 8940
   ttgcccgcag tcagcgcagg cggtgccgaa cccgtgttgg aattggacac cctcatgggc 9000
   tggttctagc ccgaattttt cgcggaatgt tgcctttggc ggtgtgtaga taagcgaaag 9060
   tgcctgtcct gcaagggata attggacttc tctacggttc aaagatcctg acgggaaggc 9120
   acttcgcagg tgaagaatat cgcggccgca tcgcgggtga aagtgtcggc cgacggccat 9180
   ggcgtcgtgt cgcatgccgg gatgggcctg ctacgtgaac tggccgatcg gaccgggtta 9240
   tcggcgcagg tcacggctgc tttggccgac acctaccgcg ggccgtgggt gtatgcgccc 9300
   ggagacgtgt tcgctgatct ggcggctgcg gttgctgacg gggcggactg catcgacggg 9360
   gtcggccagc tctgcggcga ccgtgagcac gccttcggtg cgaaagcctc gacgaccacg 9420
   atgtggcggc tggtcgatga gcgcatcgac gccgcacacc tacccgcggt gcgggcggcc 9480
   cgagcgggcg cgcgggcggc ggcctgggcc gccggtgccg cccccgctcc gggtgactgg 9540
   ctgcacatcg acatcgacgc caccctggtg atcgatcact ccgacaacaa aggcggtgcg 9600
   acgccgacct ggaagaagtc gttcgggcac catccgctgc tggcatttct ggaccgcccc 9660
   gagatcgccg gcggggaagc cctggccggg ctgctacgca ccggcaacgc cggctccaac 9720
   accgccagcg accacgtcat cgtgctggcc caagcgctgg cagcgctgcc cgcggcctgg 9780
   cgacccgacc ccagggctgg cgatcatccc gacaagccca aggtgctggt gcgctgcgac 9840
   accgccggag ccacccacac cttcgccgac gcctgccggg ccgccggggt ggggttctcc 9900
   ttcggttacc ccgtcgactg gcgcgtccaa gacgcggtgg acaccctcaa cctcgccgag 9960
   ggctggtacc cggcgatcgg caccgccggc ggtctccgcg acggcgcctg gatcgccgaa 10020
   gccaccaccc tggtcaacct gtcgtcgtgg ccaccgggca cgcggctgat cctgcgcaaa 10080
   gaaaggccgc atccgggcgc ccagctgcga ttcaccgacg ccgacgggat gcggatcaca 10140
   gcgttcatca ccgacacacc acccggcgtc gtgtcaggac aggtcgccgg cctggaactg 10200
   cgccaccgtc agcacgcccg cgtcgaagac cgcatccgcg aactcaaagc caccggcctg 10260
   cgcaacctgc cctgccactc attttgggcc aacgccgcat ggctggaaat cgccctcacc 10320
   gccgccg 10327
<210> 12
   <211> 1480
   <212> DNA
   <213> Mycobacterium austroafricanum
<400> 12
   agagtttgat tctggctcag gacgaacgct ggcggcgtgc ttaacacatg caagtcgaac 60
   ggaaaggccc ttcggggtac tcgagtggcg aacgggtgag taacacgtgg gtgatctgcc 120
   ctgcactttg ggataagcct gggaaactgg gtctaatacc gaatacaccc ttctggctgc 180
   atggtctggt tggggaaagc ttttgcggtg tgggatgggc ccgcggccta tcagcttgtt 240
   ggtgaggtta cggctcacca aggcgacgac gggtagccgg cctgagaggg tgaccggcca 300
   cactgggact gagatacggc ccagactcct acgggaggca gcagtgggga atattgcaca 360
   atgggcgcaa gcctgatgca gcgacgccgc gtgagggatg acggccttcg ggttgtaaac 420
   ctctttcgcc agggacgaag cgcaagtgac ggtacctgga gaagaaggac cggccaacta 480
   cgtgccagca gccgcggtaa tacgtagggt ccgagcgttg tccggaatta ctgggcgtaa 540
   agagctcgta ggtggtttgt cgcgttgttc gtgaaaactc acagctcaac tgtgggcgtg 600
   cgggcgatac gggcagactg gagtactgca ggggagactg gaattcctgg tgtagcggtg 660
   gaatgcgcag atatcacgag gaacaccggt ggcgaaggcg ggtctctggg cagtaactga 720
   cgctgaggag cgaaagcgtg gggagcgaac aggattagat accctggtag tccacgccgt 780
   aaacggtggg tactaggtgt gggtttcctt ccttgggatc cgtgccgtag ctaacgcatt 840
   aagtaccccg cctggggagt acggccgcaa ggctaaaact caaagaaatt gacgggggcc 900
   cgcacaagcg gcggagcatg tggattaatt cgatgcaacg cgaagaacct tacctgggtt 960
   tgacatgcac aggacgtgcc tagagatagg tattcccttg tggcctgtgt gcaggtggtg 1020
   catggctgtc gtcagctcgt gtcgtgagat gttgggttaa gtcccgcaac gagcgcaacc 1080
   cttatcttat gttgccagcg cgtaatggcg gggactcgtg agagactgcc ggggtcaact 1140
   cggaggaagg tggggatgac gtcaagtcat catgcccctt atgtccaggg cttcacacat 1200
   gctacaatgg ccggtacaaa gggctgcgat gccgtgaggt ggagcgaatc ctttcaaagc 1260
   cggtctcagt tcggatcggg gtctgcaact cgaccccgtg aagtcggagt cgctagtaat 1320
   cgcagatcag caacgctgcg gtgaatacgt tcccgggcct tgtacacacc gcccgtcacg 1380
   tcatgaaagt cggtaacacc cgaagccggt ggcctaaccc cttgtgggag ggagccgtcg 1440
   aaggtgggat cggcgattgg gacgaagtcg taacaaggta 1480
<210> 13
   <211> 1362
   <212> DNA
   <213> Mycobacterium austroafricanum
<400> 13
   tcgaacggaa aggcccttcg gggtactcga gtggcgaacg ggtgagtaac acgtgggtga 60
   tctgccctgc actttgggat aagcctggga aactgggtct aataccgaat acacccttct 120
   ggctgcatgg tctggttggg gaaagctttt gcggtgtggg atgggcccgc ggcctatcag 180
   cttgttggtg aggttacggc tcaccaaggc gacgacgggt agccggcctg agagggtgac 240
   cggccacact gggactgaga tacggcccag actcctacgg gaggcagcag tggggaatat 300
   tgcacaatgg gcgcaagcct gatgcagcga cgccgcgtga gggatgacgg ccttcgggtt 360
   gtaaacctct ttcgccaggg acgaagcgca agtgacggta cctggagaag aaggaccggc 420
   caactacgtg ccagcagccg cggtaatacg tagggtccga gcgttgtccg gaattactgg 480
   gcgtaaagag ctcgtaggtg gtttgtcgcg ttgttcgtga aaactcacag ctcaactgtg 540
   ggcgtgcggg cgatacgggc agactggagt actgcagggg agactggaat tcctggtgta 600
   gcggtggaat gcgcagatat caggaggaac accggtggcg aaggcgggtc tctgggcagt 660
   aactgacgct gaggagcgaa agcgtgggga gcgaacagga ttagataccc tggtagtcca 720
   cgccgtaaac ggtgggtact aggtgtgggt ttccttcctt gggatccgtg ccgtagctaa 780
   cgcattaagt accccgcctg gggagtacgg ccgccagggc taaaactcaa agaaattgac 840
   gggggcccgc acaagcggcg gagcatgtgg attaattcga tgcaacgcga agaaccttac 900
   ctgggtttga catgcacagg acgtgcctag agataggtat tcccttgtgg cctgtgtgca 960
   ggtggtgcat ggctgtcgtc agctcgtgtc gtgagatgtt gggttaagtc ccgcaacgag 1020
   cgcaaccctt atcttatgtt gccagcgcgt aatggcgggg actcgtgaga gactgccggg 1080
   gtcaactcgg aggaaggtgg ggatgacgtc aagtcatcat gccccttatg tccagggctt 1140
   cacacatgct acaatggccg gtacaaaggg ctgcgatgcc gtgaggtgga gcgaatcctt 1200
   tcaaagccgg tctcagttcg gatcggggtc tgcaactcga ccccgtgaag tcggagtcgc 1260
   tagtaatcgc agatcagcaa cgctgcggtg aatacgttcc cgggccttgt acacaccgcc 1320
   cgtcacgtca tgaaagtacg gtaacacccg aagccggtgg ta 1362
<210> 14
   <211> 591
   <212> DNA
   <213> Mycobacterium austroafricanum
<400> 14
   acggtctcgt cggcaaatac ctgatgcacc acattcttcg cgttctggcg agcgtggttc 60
   gcacaagccg gatggaaggc tacaagggag ccttcggcgc tccactgtat tcgcacgagt 120
   tctaccacac cgacaccaat cgcggcttcg tcaacggttt cggcatgcag gtggcacgca 180
   gcttcggcgc tgcatacaca gcaatgggca gccacaccgg ttacgtggcc ccctggggca 240
   aatcgcatcg caagttcttc aacgaacact tcggcaatca cttgatggtt ttcatgttcg 300
   gcgaagacct ccccgtcgag acgaactgcg tgacactcga tcctgacgcc aaagactcga 360
   gtggcctccc tgcggcgcgt gtcaactggg aaccacacga gaacgacatc gcgctggcca 420
   attacggcat cgaccggatc ttcgaggccg cgcgagcgtt gggcgctgtc gagaccaacg 480
   acaccggcgt gctcaatcct ccgcccggtt ggcacttgat gggcacctgt cggatgggta 540
   acaacccaga agattcggtc accaacaagt ggcatcagac ctgggatgtg c 591
<210> 15
   <211> 20
   <212> DNA
   <213> artificial
<220>
   <223> primer Bott1
<400> 15
   tgcacacagg ccacaaccca 20
<210> 16
   <211> 21
   <212> DNA
   <213> artificial
<220>
   <223> primer Bott2
<400> 16
   gagagtttga tcctggctca g 21
<210> 17
   <211> 20
   <212> DNA
   <213> artificial
<220>
   <223> primer 244
<400> 17
   cccactgctg cctcccgtag 20
<210> 18
   <211> 20
   <212> DNA
   <213> artificial
<220>
   <223> primer Tb11
<400> 18
   accaacgatg gtgtgtccat 20
<210> 19
   <211> 20
   <212> DNA
   <213> artificial
<220>
   <223> primer Tb12
<400> 19
   cttgtcgaac cgcataccct 20
<210> 20
   <211> 24
   <212> DNA
   <213> artificial
<220>
   <223> primer MaFV2
<400> 20
   gtctaatacc gaatacaccc ttct 24
<210> 21
   <211> 24
   <212> DNA
   <213> artificial
<220>
   <223> primer MaRV6
<400> 21
   gtagttggcc ggtccttctt ctcc 24
<210> 22
   <211> 20
   <212> DNA
   <213> artificial
<220>
   <223> primer MF1
<400> 22
   tgagaagcct cgtgtattac 20
<210> 23
   <211> 18
   <212> DNA
   <213> artificial
<220>
   <223> primer MR1
<400> 23
   gagataaggc gtggtgaa 18
<210> 24
   <211> 20
   <212> DNA
   <213> artificial
<220>
   <223> primer MF2
<400> 24
   agtgacggca cccataagtg 20
<210> 25
   <211> 21
   <212> DNA
   <213> artificial
<220>
   <223> primer MR2
<400> 25
   tcgaggtgtt gaggtccgaa t 21
<210> 26
   <211> 18
   <212> DNA
   <213> artificial
<220>
   <223> primer MF3
<400> 26
   atcatcccgt ggaactac 18
<210> 27
   <211> 18
   <212> DNA
   <213> artifïcial
<220>
   <223> primer MR3
<400> 27
   tgacctgggc gatgtgtt 18
<210> 28
   <211> 18
   <212> DNA
   <213> artificial
<220>
   <223> primer MF4
<400> 28
   atcagacctg ggatgtgc 18
<210> 29
   <211> 19
   <212> DNA
   <213> artificial
<220>
   <223> primer MR4
<400> 29
   ggctgtgaaa gtcggatga 19
<210> 30
   <211> 20
   <212> DNA
   <213> artificial
<220>
   <223> primer RT-PCR-F1
<400> 30
   agtgacggca cccataagtg 20
<210> 31
   <211> 21
   <212> DNA
   <213> artificial
<220>
   <223> primer RT-PCR-R1
<400> 31
   tcgaggtgtt gaggtccgaa t 21
<210> 32
   <211> 20
   <212> DNA
   <213> artificial
<220>
   <223> primer RT-PCR-F2
<400> 32
   gcaggtcggc tcggtaatga 20
<210> 33
   <211> 20
   <212> DNA
   <213> artificial
<220>
   <223> primer RT-PCR-R2
<400> 33
   gtaatacacg aggcttctca 20
<210> 34
   <211> 20
   <212> DNA
   <213> artificial
<220>
   <223> primer RT-PCR-F3
<400> 34
   acggtctcgt cggcaaatac 20
<210> 35
   <211> 18
   <212> DNA
   <213> artificial
<220>
   <223> primer RT-PCR-R3
<400> 35
   gcacatccca ggtctgat 18

## Description

La présente demande est relative au domaine de la microbiologie, et plus particulièrement à l'utilisation de microorganismes pour traiter des effluents contaminés par des polluants chimiques, notamment le méthyl *tert*-butyl éther (MTBE) et/ou des intermédiaires cataboliques de ce composé.

La présente invention concerne de nouveaux polypeptides et leurs fragments, ainsi que les acides nucléiques codants lesdits polypeptides isolés de microorganismes capables de métaboliser le MTBE, et/ou l'un au moins des intermédiaires cataboliques du MTBE, préférentiellement le *tert*-butyl alcool (TBA), le 2-méthyl 1,2-propanediol (2-M1,2-PD), l'hydroxyisobutyraldéhyde, l'acide hydroxyisobutyrique (HIBA). L'invention concerne également des vecteurs de clonage et/ou d'expression comprenant lesdits acides nucléiques, des cellules bactériennes transformées par lesdits acides nucléiques ou lesdits vecteurs ainsi que leurs utilisations.

L'invention concerne également un procédé d'identification de microorganismes capables de métaboliser le MTBE, et/ou l'un au moins des intermédiaires cataboliques du MTBE. Plus particulièrement, la présente invention concerne une nouvelle souche bactérienne *Mycobacterium austroafricanum* enregistrées dans la collection CNCM sous le numéro I-3401.

L'invention concerne également un procédé de traitement d'effluents contaminés par le MTBE et/ou l'un au moins des intermédiaires cataboliques du MTBE.

Le composé chimique MTBE est utilisé comme additif aux essences sans plomb. Depuis que l'utilisation des alkyles de plomb est interdite en raison de leur toxicité, le MTBE est ajouté dans les essences afin d'augmenter leur indice d'octane. L'indice d'octane, mesure la "résistance au cliquetis" des carburants lorsqu'ils brûlent en mélange avec l'air dans la chambre de combustion des moteurs. Le MTBE est également utilisé en tant que composé oxygéné permettant d'augmenter la teneur en oxygène des essences et, de ce fait, d'améliorer leur efficacité de combustion. Ceci permet de réduire le rejet en hydrocarbures imbrûlés ainsi qu'en monoxyde de carbone dans l'atmosphère. Ainsi, des concentrations en MTBE de 15% (v/v) sont couramment utilisées dans les carburants oxygénés. Le MTBE a été classé comme composé cancérigène potentiel par l'agence américaine pour la protection de l'environnement (US E.P.A., December 1997 EPA/822/F-97/008. Office of Water, Washington, DC, USA). La contamination de ce composé dans l'environnement peut être due au stockage inadéquat de l'essence dans des réservoirs non étanches ou des déversements accidentels. Ce type de déchets peut causer de sérieux problèmes de pollution environnementale tels que la contamination des aquifères souterrains. Les consommateurs peuvent être exposés à de faibles concentrations lorsqu'ils boivent une eau non potable provenant d'une source contaminée par le MTBE. Par ailleurs, le goût et l'odeur déplaisants que le MTBE confère à l'eau, même à de faibles concentrations, rend celle-ci impropre à la consommation, faisant de ce composé xénobiotique un polluant important. La contamination du MTBE semble être largement attribuable au fait qu'il soit difficilement éliminé de l'environnement. Le MTBE s'avère persistant du fait de sa solubilité élevée dans l'eau et sa faible biodégradabilité. La demi-vie du MTBE dans les aquifères est estimée à au moins 2 ans (Wilson J. T., 2003, 19-61 : In E. E. Moyer and P. T. Kostecki (ed.), MTBE Remediation Handbook. Amherst Scientific Publishers, Amherst, MA.) ; cette valeur peut être comparée à celle du benzène qui est de 2 à 3 mois dans des conditions identiques. La contamination des aquifères peut engendrer des risques sérieux pour la santé publique. Il est donc nécessaire de développer des procédés efficaces permettant de traiter les aquifères contaminés par le MTBE ou tout autre intermédiaire catabolique du MTBE.

Plusieurs études visant à déterminer la biodégradabilité du MTBE ont été entreprises depuis quelques années. Un certain nombre de microorganismes capables d'assimiler complètement ou partiellement le MTBE ont été identifiés et isolés. Par exemple, certains microorganismes sont capables de cataboliser le MTBE par cométabolisme. Deux voies de dégradation, impliquant toutes deux l'oxydation initiale du MTBE, conduisent à la production de l'alcool tert-butylique (TBA) qui, le plus généralement, s'accumule dans le milieu. La souche bactérienne Mycobacterium vaccae JOB5, lorsqu'elle est cultivée sur du propane, est capable d'oxyder le TBA bien que cela ne lui permette pas de générer des composés utiles pour sa croissance. Quelques souches de microorganismes capables d'assimiler le MTBE comme source de carbone et d'énergie ont pu être isolées (François et al., Appl. Environ. Microbiol., 2002, 68: 2754-2762; Hanson, J. R., et al. Appl. Environ. Microbiol., 1999. 65: 4788-4792; Hatzinger, P. B., et al., Appl. Environ. Microbiol., 2001, 67: 5601-560). Les mécanismes enzymatiques de l'attaque initiale du MTBE chez ces bactéries n'ont pas été encore élucidés.

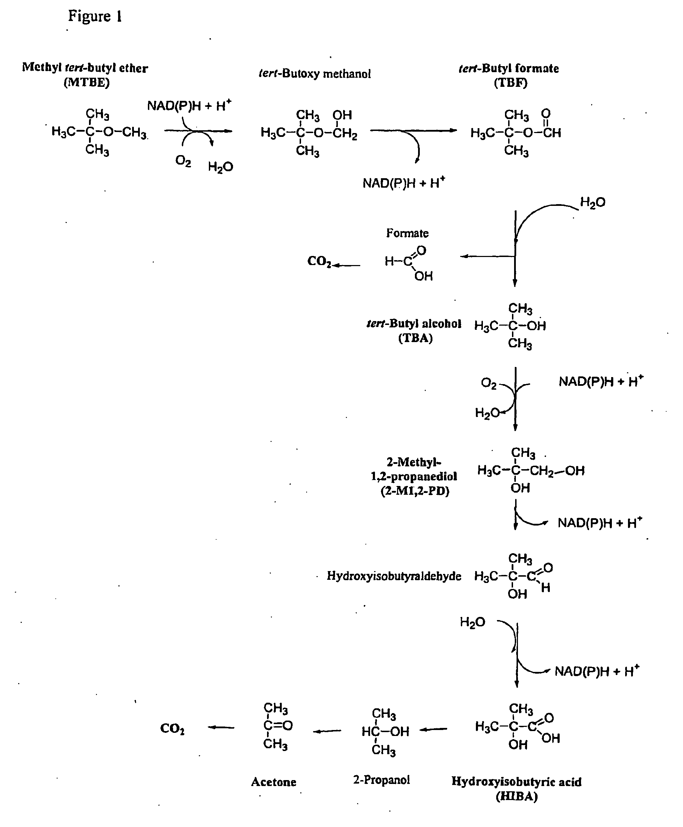

La souche bactérienne *Mycobacterium austroafricanum* I-2562 est une des bactéries connues pour sa capacité à croître en conditions aérobies, en présence de MTBE. Cette souche est en effet capable de cataboliser le MTBE en source de carbone et d'énergie (François et al., Appl. Environ. Microbiol., 2002, 68: 2754-2762 ; US patent n° 6,849,445). Un certain nombre d'intermédiaires de dégradation ou cataboliques du MTBE ont été identifiés, tels que le TBA, le 2 M1,2-PD, l'hydroxyisobutyraldéhyde et le HIBA. La voie de dégradation du MTBE par cette souche bactérienne est décrite à la figure 1.

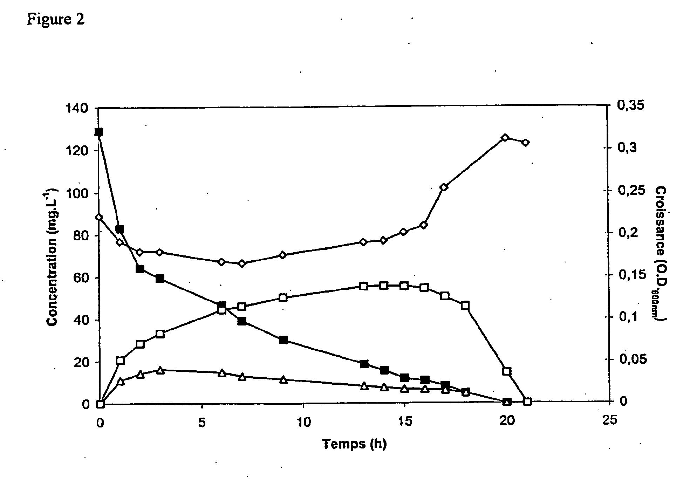

Les cinétiques de dégradation du MTBE et de la croissance qui l'accompagne chez *M. austroafricanum* I-2562 présentent des phases bien distinctes (Figure 2). La cinétique de dégradation du MTBE est caractérisée par une vitesse de dégradation très rapide au cours des premières 48 h, qui diminue du 2^{ème} au 16^{ème} jour. Pendant cette étape de transformation du MTBE en TBA, le TBA s'accumule et aucune croissance n'est observée. Du 16^{ème} au 21^{ème} jour, le TBA accumulé est dégradé, induisant la croissance du microorganisme.

Il est à noter que la première étape de dégradation comprend la minéralisation du formiate en CO₂ nécessitant que le microorganisme soit méthylotrophe (François et al., Appl. Environ. Microbiol., 2002, 68: 2754-2762). La formiate déshydrogénase NAD(P)⁺-dépendante (ou FDH) est une enzyme qui joue un rôle important dans la production d'énergie chez les microorganismes méthylotrophes. Cette enzyme est, d'ailleurs, fréquemment utilisée dans la régénération de cofacteur de type NAD(P)⁺ dans des réactions de biocatalyse (Tishkov, V. I. et al. Biochemistry (Moscow), 2004, 69: 1537-1554). Cette première étape du catabolisme ne génère pas d'ATP. Francois et al. ont montré que le TBF avait un effet négatif sur la vitesse de dégradation du MTBE chez *M. austroafricanum* I-2562 (François et al., Appl. Microbiol. Biotechnol. 2003, 62: 256-262). Il a été suggéré que le déficit en équivalents réduits de coenzymes et en énergie était responsable du ralentissement des réactions enzymatiques (Salanitro, Curr. Op. Biotechnol., 1995, 6: 337-340). Ces deux raisons sont très probablement principalement responsables du ralentissement de la vitesse de dégradation du MTBE.

La seconde étape, en permettant la production nette de 2 NAD(P)H et de 2H⁺, alimente ainsi la chaîne de transport d'électrons avec l'O₂ comme accepteur final d'électrons en aérobiose. Cette étape apparaît donc essentielle dans le métabolisme du MTBE. Le résultat final de cette chaîne de transport d'électrons est de régénérer du NAD(P)⁺ à partir du NAD(P)H, de réduire l'O₂ en H₂O, créant un gradient de transport des protons qui sont utilisés pour la formation de l'ATP par l'ATP synthase (pompe à protons). Ceci correspond au mécanisme de phosphorylation oxydative et permet la synthèse d'ATP qui est le composé énergétique des microorganismes (30,5 kJ produit/mole d'ATP dégradé du fait d'une liaison à haut niveau énergétique).

Un objectif de l'invention est de disposer de polypeptides capables de métaboliser le MTBE, et/ou l'un au moins des intermédiaires cataboliques du MTBE, préférentiellement le TBA, le 2-M1,2-PD, l'hydroxyisobutyraldéhyde, le HIBA, éventuellement de les modifier et de les utiliser afin de traiter des effluents contaminés par le MTBE, et/ou au moins l'un des intermédiaires cataboliques du MTBE, préférentiellement le TBA, le 2-M1,2-PD, l'hydroxyisobutyraldéhyde, le HIBA.

Les travaux de François et al. ont permis de mettre en évidence que plusieurs polypeptides étaient spécifiquement induits en présence de MTBE ou TBA chez M. austroafricanum IFP 2012 (I-2562). Ces polypeptides spécifiquement induits ont été purifiés, digérés avec de la trypsine puis microséquencés. L'analyse des séquences résultantes à l'aide des outils d'alignement Blast et Fasta a suggéré que ces polypeptides correspondent à des oxidoréductases impliquées dans la dégradation du MTBE et du TBA.

La Demanderesse a réalisé de nouveaux séquençages peptidiques à partir de polypeptides spécifiquement induits en présence de MTBE, de manière à obtenir une première sonde de 204 paires de bases. Cette sonde de 204 paires de bases s'est cependant révélée trop courte pour une hybridation sur colonies. De nombreux alignements BLAST ont donc été réalisés avec les protéines les plus similaires à la séquence en acides aminés déduite du fragment d'ADN de 204 pb. Après identification des séquences en acides aminés fortement conservées sur toutes ces protéines, de nombreuses amplifications PCR ont été réalisées en utilisant des amorces dégénérées, permettant d'obtenir une nouvelle sonde de 604 paires de bases. Cette sonde de 604 paires de bases a permis de réaliser le clonage d'un fragment d'ADN comprenant le gène mpdC, l'orf1, le gène mpdB, l'orf2 et une séquence correspondant à une transposase putative. Le clonage de ce fragment d'ADN s'est révélé difficile, puisqu'à la suite d'un premier clonage, les clones obtenus après transformation dans E. Coli n'étaient pas stables, ce qui rendait problématique l'extraction de l'ADN plasmidique. Après de nombreuses expériences, la Demanderesse a fait l'hypothèse que l'instabilité des clones obtenus provenait de l'expression du gène transposase, et a donc cloné un fragment d'ADN dans lequel le gène de la transposase est supprimé et qui correspond à la séquence SEQ ID N°11. Ainsi des clones stables comprenant la séquence SEQ ID N°11 ont pu être obtenus.

La Demanderesse a ainsi du faire face à de nombreuses difficultés de clonage et séquençage pour obtenir les polypeptides conformes à l'invention.

La présente invention vise tout polypeptide isolé ou purifié ayant une activité dans la voie de dégradation du MTBE et/ou l'un au moins des intermédiaires cataboliques du MTBE, préférentiellement le TBA, le 2 M1,2-PD, l'hydroxyisobutyraldéhyde, le HIBA.

En précisant que les polypeptides ou les acides nucléique codant pour au moins un polypeptides conforme à l'invention sont « isolés ou purifiés », on indique qu'ils sont placés dans un environnement différent de celui dans lequel ils se trouvent naturellement. Ils peuvent être isolés ou purifiés d'une souche bactérienne capable de croître dans un milieu comprenant du MTBE et/ou l'un au moins des intermédiaires cataboliques du MTBE, préférentiellement sélectionnés dans le groupe consistant en le TBA, le 2-M1,2-PD, l'hydroxyisobutyraldéhyde, le HIBA, dont notamment les souches *Mycobacterium austroafricanum* enregistrées dans la collection CNCM sous les numéros I-3401 et I-2532. On entend également les molécules qui ont été altérées par la présente invention comme étant « isolées ». On rappelle que ces qualificatifs d' « isolé ou purifié » sont également utilisés pour les cellules hôtes.

Dans le contexte de l'invention, les termes « polypeptide, enzyme » et « protéine » peuvent être utilisés de façon interchangeable et désignent des molécules caractérisées par des séquences en acides aminés de toute longueur, éventuellement modifiées par voie chimique ou biochimique. On inclut également dans le terme « polypeptide » l'ensemble des polypeptides mutés pouvant exister naturellement ou variants, en particulier chez la bactérie de la souche *M. austroafricanum,* et qui correspondent à des substitutions, délétions, insertions ou additions d'au moins un acide aminé. Dans le cas d'une substitution, un ou plusieurs acides aminés consécutifs ou non consécutifs, peuvent être remplacés par des acides aminés « équivalents ». L'expression acide aminé « équivalent » vise ici à désigner tout acide aminé susceptible d'être substitué à l'un des acides aminés de la structure de base sans cependant modifier l'activité biologique des polypeptides selon l'invention. Ces acides aminés équivalents peuvent être déterminés soit en s'appuyant sur leur homologie de structure avec les acides aminés auxquels ils se substituent, soit sur les résultats des essais d'activité biologique croisée auxquels les différents polypeptides sont susceptibles de donner lieu.

Le polypeptide selon l'invention est sélectionné dans le groupe consistant en :
a) un polypeptide comprenant une séquence en acides aminés sélectionnée dans le groupe consistant en SEQ ID NO :2ou SEQ ID NO: 10,
b) un polypeptide comprenant une séquence en acides aminés qui présente au moins 70 % d'identité, préférentiellement 75%, 80%, 90%, 95%, 98% ou 99% d'identité avec la séquence en acides aminés d'un polypeptide comprenant SEQ ID NO :2, le polypeptide tel que défini en a) ou b) de séquence SEQ ID NO :2 ayant une activité de déshydrogénation de l'hydroxyisobutyraldéhyde en HIBA, et
le polypeptide tel que défini en a) de séquence SEQ ID NO: 10 ayant une activité de régulateur transcriptionnel..

Différents protocoles connus de l'homme de l'art ont été décrits pour introduire des mutations dans les polypeptides. Typiquement, pour modifier la séquence en acides aminés d'un polypeptide, on agit sur la molécule d'acide nucléique qui code pour ce polypeptide. Il est possible pour modifier l'acide nucléique codant pour un polypeptide conforme à l'invention, de le soumettre à un traitement par un agent mutagène, c'est-à-dire d'un agent physique ou chimique capable de provoquer des mutations qui altèrent la signification des codons, ce qui par le jeu du code génétique modifie la séquence en acides aminés. Avantageusement, on a recours à une technique de mutagénèse dirigée pour modifier la séquence nucléotidique d'un acide nucléique codant pour au moins un polypeptide du gène ainsi que celle des polynucléotides conformes à l'invention; il s'agit alors d'introduire spécifiquement une ou plusieurs mutations dans l'acide nucléique étudié, ce qui par le jeu du code génétique entraîne la substitution d'un ou plusieurs acides aminés par un ou plusieurs autres acides aminés dans le polypeptide codé par le polynucléotide muté. L'intérêt de réaliser ces mutations est non seulement d'étudier l'activité biologique d'un des polypeptides conformes à l'invention dans la voie de dégradation du MTBE et/ou l'un au moins des intermédiaires catalytiques, mais éventuellement d'optimiser ladite activité du polypeptide sous forme recombinante en vue de son application industrielle.

Par « fragment de polypeptide », on désigne un polypeptide comportant au minimum 15 acides aminés consécutifs, de préférence 17, 20, 23, 25, 30, 40, 50, 100, 250 ou 300 acides aminés consécutifs. Les fragments de polypeptide conforme à l'invention peuvent être obtenus par clivage dudit polypeptide par une enzyme protéolytique, par un réactif chimique, ou encore en plaçant ledit polypeptide dans un environnement très acide font également partie de l'invention.

Par « fragment biologiquement actif », on entend un fragment de séquences en acides aminés d'un polypeptide conforme à l'invention ayant au moins une des caractéristiques ou propriétés fonctionnelles dudit polypeptide, notamment en ce qu'il a une activité dans la voie de dégradation du MTBE et/ou l'un au moins des intermédiaires cataboliques du MTBE, préférentiellement le TBA, le 2-M1,2-PD, l'hydroxyisobutyraldéhyde et le HIBA.

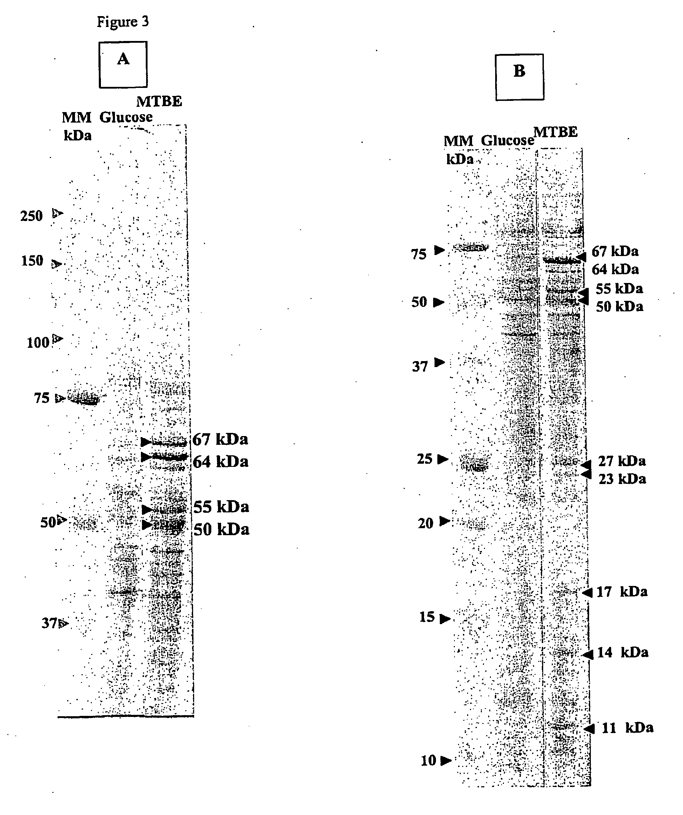

Selon un mode préféré de réalisation, l'expression des polypeptides conformes à l'invention est induite lorsque les bactéries susceptibles d'assimiler le MTBE et/ou au moins l'un des intermédiaires cataboliques du MTBE, dont notamment les bactéries de la souche *M. austroafricanum,* plus préférentiellement les bactéries I-2562 ou I-3401, sont dans un milieu comprenant du MTBE et/ou au moins l'un des intermédiaires cataboliques du MTBE, préférentiellement le TBA, le 2-M1,2-PD, l'hydroxyisobutyraldéhyde et le HIBA. Préférentiellement, les polypeptides dont l'expression est fortement induite sont les polypeptides SEQ ID NO :2 tel que défini en a) ou b), SEQ ID NO:4 et SEQ ID NO :6, comme indiqué sur la figure 3.

Avantageusement, le polypeptide SEQ ID NO :2 tel que défini en a) ou b) et dénommé MpdC a une activité d'aldéhyde déshydrogénase. Dans la voie catabolique du MTBE, le polypeptide MpdC conforme à l'invention est préférentiellement susceptible de déshydrogéner l'hydroxyisobutyraldéhyde en HIBA.

Avantageusement, le polypeptide SEQ ID NO:6 dénommé MpdB a une activité d'alcool déshydrogénase. Dans la voie catabolique du MTBE, le polypeptide MpdB conforme à l'invention est préférentiellement susceptible de déshydrogéner le 2-M1,2-PD en hydroxyisobutyraldéhyde.

Avantageusement, le polypeptide SEQ ID NO:8 a une activité de perméase, préférentiellement de di/tripeptide perméase. Ledit polypeptide comprend préférentiellement 5 segments transmembranaires.

Avantageusement, le polypeptide SEQ ID NO:10 tel que défini en a) et dénommé MpdR a une activité de régulateur transcriptionnel.

La présente invention vise également tout acide nucléique purifié ou isolé codant pour au moins un polypeptide conforme à l'invention. Préférentiellement, la présente invention a pour objet tout acide nucléique isolé ou purifié codant pour au moins un polypeptide ayant une activité dans la voie de dégradation du MTBE, ou l'un au moins des intermédiaires cataboliques du MTBE, préférentiellement le TBA, le 2-M1,2-PD, l'hydroxyisobutyraldéhyde et le HIBA.

Dans le contexte de l'invention, les termes « acide nucléique » et « ADN » sont utilisés de façon interchangeable. Par « acide nucléique », on désigne un enchaînement précis de nucléotides, modifiés ou non, permettant de définir un fragment ou une région d'un acide nucléique, comportant ou non des nucléotides non naturels, et pouvant correspondre aussi bien à un ADN double brin, un ADN simple brin que des produits de transcription desdits ADN, et/ou un fragment d' ARN.

L'acide nucléique selon l'invention est sélectionné dans le groupe consistant en :
e) un acide nucléique comprenant au moins l'une quelconque des séquences nucléotidiques sélectionnée dans le groupe consistant en SEQ ID NO:1 ou SEQ ID NO:9, ou complément de celle-ci,
f) un acide nucléique comprenant au moins une séquence nucléotidique qui présente au moins 70 % d'identité, préférentiellement 75%, 80%, 90%, 95%, 98% ou 99% d'identité avec la séquence nucléotidique d'un acide nucléique tel que défini en e).

Par « complément », on désigne tout acide nucléique dont les nucléotides sont complémentaires de ceux de la séquence SEQ ID NO:1 ou SEQ ID NO:9, et dont l'orientation est inversée.

Par « pourcentage d'identité » entre deux séquences d'acides nucléiques ou d'acides aminés au sens de la présente invention, on désigne un pourcentage de nucléotides ou d'acides aminés identiques entre les deux séquences à comparer. Le pourcentage d'identité entre deux séquences d'acides nucléiques ou d'acides aminés est déterminé en comparant ces deux séquences alignées de manière significative, la séquence d'acides nucléiques ou d'acides aminés à comparer pouvant comprendre des additions ou des délétions par rapport à la séquence de référence pour un alignement significatif entre ces deux séquences. Le pourcentage d'identité est calculé en déterminant le nombre de positions identiques pour lesquelles le nucléotide ou l'acide aminé est identique entre les deux séquences, en divisant ce nombre de positions identiques par le nombre total de positions comparées et en multipliant le résultat obtenu par 100 pour obtenir le pourcentage d'identité entre ces deux séquences. Par « alignement significatif », on désigne l'alignement pour lequel le pourcentage d'identité déterminé comme ci-après est le plus élevé. Préférentiellement, on utilisera le programme BLAST pour obtenir un alignement significatif.

Avantageusement, l'acide nucléique conforme à l'invention, comprend les séquences nucléotidiques SEQ ID NO:1 telle que définie en e) ou f) et SEQ ID NO:5, préférentiellement SEQ ID NO:1 telle que définie en e) ou f), SEQ ID NO:3 et SEQ ID NO:5.

Avantageusement, l'acide nucléique conforme à l'invention comprend la séquence nucléotidique SEQ ID NO :11.

Selon un mode préféré de réalisation, la transcription de l'acide nucléique conforme à l'invention est sous le contrôle d'un promoteur unique. Par « promoteur », on désigne une région de régulation située en amont d'un cadre de lecture ouvert (ORF), à proximité de son extrémité 5'. Dans la présente demande, on utilisera les termes « ORF » et « gène » de façon interchangeable. Un promoteur comporte certaines séquences nucléotidiques caractéristiques permettant la fixation du complexe d'initiation de la transcription ainsi que la fixation de régulateurs transcriptionnels.

Selon un autre mode préféré de réalisation, l'acide nucléique conforme à l'invention est caractérisé en ce que sa transcription est induite par la présence de MTBE, et/ou l'un au moins des intermédiaires cataboliques du MTBE, préférentiellement sélectionnés dans le groupe consistant en le TBA, le 2-M1,2-PD, l'hydroxyisobutyraldéhyde, le HIBA. Avantageusement, l'acide nucléique conforme à l'invention est organisé en opéron.

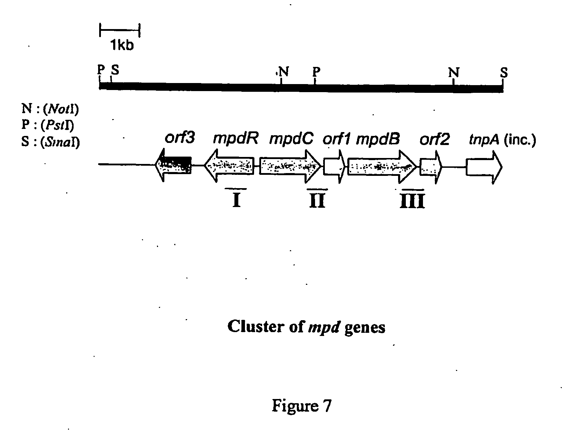

Selon un mode préféré de réalisation, l'acide nucléique conforme à l'invention correspond à un cluster comprenant les acides nucléiques codant pour SEQ ID NO:1, SEQ ID NO :3, SEQ ID NO :5, SEQ ID NO :7 et SEQ ID NO :9, ledit cluster étant alors dénommé cluster *mpd.* Le cluster *mpd* est schématisé à la figure 7.

Selon un mode préféré de réalisation, l'acide nucléique conforme à l'invention est un acide nucléique recombinant. Par « acide nucléique recombinant », on désigne une molécule d'acide nucléique, simple ou double brin, qui a été modifiée par une intervention humaine de manière à contenir des fragments d'acides nucléiques combinés ou juxtaposés selon un arrangement n'existant pas à l'état naturel.

L'invention a également pour objet tout vecteur d'expression et/ou de clonage comprenant un acide nucléique conforme à l'invention. Par « vecteur », on désigne une molécule d'acide nucléique extrachromosomique, notamment un plasmide, ayant une réplication autonome et pouvant être incorporé dans une « cellule hôte ».

Typiquement, un vecteur de clonage est conçu pour permettre le transport d'un fragment d'ADN cloné et contient un ou plusieurs sites de reconnaissance pour des enzymes de restriction qui permettent l'insertion ou clonage d'un fragment d'acide nucléique ainsi qu'une ou des séquences nucléotidiques codantes pour des gènes permettant l'identification et la sélection de cellules hôtes transformées par ledit vecteur tels que des gènes de résistance à des antibiotiques.

Selon un mode préféré de réalisation, le vecteur d'expression conforme à l'invention est conçu pour permettre l'expression d'une séquence nucléotidique codante insérée en aval d'un promoteur. La séquence insérée ou insert est alors transcrite, puis traduite en polypeptide. Avantageusement, le vecteur conforme à l'invention est flanqué d'éléments assurant l'expression d'au moins un acide nucléique conforme à l'invention dans la cellule hôte. Avantageusement, certains vecteurs d'expression ont une séquence codante pour un tag en amont ou en aval du site d'insertion ou de clonage ; l'acide nucléique inséré est alors transcrit puis traduit sous la forme d'une protéine de fusion. Par « protéine de fusion », on désigne un polypeptide hybride comprenant un polypeptide codé par un acide nucléique conforme à l'invention et un polypeptide capable de se fixer à des matrices d'affinité et/ou d'être reconnu par des anticorps permettant de détecter et/ou de purifier le polypeptide auquel il est fusionné.

Selon un autre mode préféré de réalisation, le vecteur conforme à l'invention comprend au moins une séquence homologue d'une séquence d'acide nucléique présente dans le génome d'une cellule hôte et assurant son intégration dans ledit génome.

L'invention concerne en outre une cellule hôte isolée comprenant soit au moins l'un des acides nucléiques conforme à l'invention et/ou au moins l'un des vecteurs conforme à l'invention. Parmi les cellules hôtes utilisables au sens de la présente invention, on peut citer une cellule procaryote, préférentiellement une cellule bactérienne, plus préférentiellement une cellule bactérienne sélectionnée dans le groupe consistant en *Mycobacterium smegmatis* mc2 155, *Escherichia Coli, Rhodococcus ruber* (enregistrée dans la collection CNCM sous le nom *Gordonia terrae* et le numéro CIP I-1885).

Les polypeptides conformes à l'invention peuvent être préparés et/ou obtenus par toutes techniques maîtrisées par l'homme de l'art. Ils peuvent notamment être obtenus par synthèse chimique mais également par des techniques de biologie moléculaire, utilisant notamment la PCR, des vecteurs d'expression et des cellules hôtes appropriés tels que décrits ci-dessus. Préférentiellement, les polypeptides conformes à l'invention se caractérisent en ce que leur expression dans des cellules hôtes, notamment des cellules bactériennes, permet de satisfaire à l'exigence de dégradation du MTBE, et/ou l'un au moins des intermédiaires cataboliques et permettent la croissance desdites bactéries.

La séquence SEQ ID NO :12 correspond à la séquence nucléotidique de l'ADNr 16S de la souche *M. austroafricanum* I-2532.

Par « sonde » on entend un fragment d'acide nucléique marqué par l'incorporation d'atomes radioactifs ou de groupements fluorescents et dont la séquence est sensiblement complémentaire à la séquence d'un acide nucléique recherché ; ce dernier sera décelé par hybridation avec la sonde, cette hybridation se produisant lorsque les deux séquences complémentaires s'apparient. Par « séquence complémentaire » on désigne une séquence nucléotidique constituée d'une succession de bases complémentaires à celle d'une autre séquence avec laquelle elle est donc capable de s'hybrider. Préférentiellement, l'hybridation d'une sonde dont la taille est supérieure à 200 nucléotides est réalisée à une température d'environ 60°C selon l'enseignement adapté de Sambrook et al. (Molecular Cloning: A Laboratory Manual. Cold Spring Harbor Laboratory Press, Cold Spring Harbor, New York. 2001).

La présente invention vise également un procédé d'identification d'une cellule ou d'un acide nucléique d'une cellule susceptible de dégrader du MTBE et/ou au moins l'un des intermédiaires cataboliques du MTBE, préférentiellement sélectionné dans le groupe consistant en le TBA, le 2-M1,2-PD, l'hydroxyisobutyraldéhyde, le HIBA, comprenant :
- optionnellement une étape d'ensemencement de la cellule sur un milieu supplémenté en MTBE, et/ou au moins l'un des intermédiaires cataboliques du MTBE, préférentiellement sélectionné dans le groupe consistant en le TBA, le 2-M1,2-PD, l'hydroxyisobutyraldéhyde, le HIBA,
- une étape de criblage des acides nucléiques de ladite cellule par hybridation avec au moins une sonde, ladite sonde comprenant la séquence nucléotidique complémentaire d'un fragment d'au moins 50 nucléotides consécutifs de la séquence nucléotidique sélectionnée dans le groupe consistant en SEQ ID NO :1 ou SEQ ID NO :9, ou ladite sonde comprenant un fragment du gène mpdB de 591 paires de bases ayant pour séquence la séquence SEQ ID n°: 14.

Selon un mode préféré de réalisation, une cellule d'une nouvelle souche capable de dégrader le MTBE, et/ou au moins l'un des intermédiaires cataboliques du MTBE, sera identifiée grâce à des amorces oligonucléotidiques permettant l'amplification de l'ARNr 16S de ladite cellule.

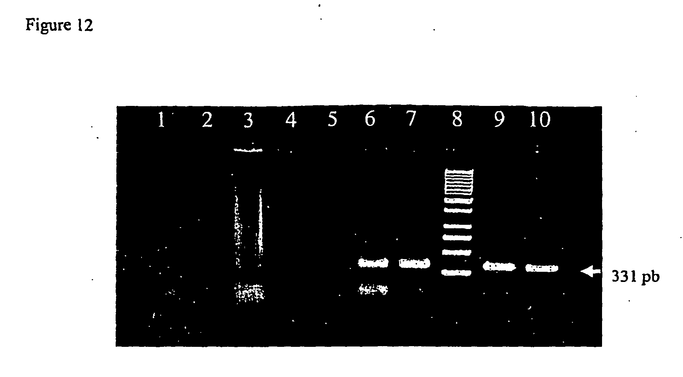

Avantageusement, un couple d'amorces (5'-TGCACACAGGCCACAACCCA-3') et (5'-GAGAGTTTGATCCTGGCTCAG-3') a été conçu à partir de deux régions variables de la séquence nucléotidique de l'ADNr 16S de la souche *M. austroafricanum.* Ce couple d'amorces est spécifique de l'espèce *M*. *austroafricanum* dans la mesure où il ne permet pas d'amplifier l'ADNr 16S de microorganismes d'autres espèces, dont notamment l'espèce des *Nocardiacae,* incapables d'assimiler le MTBE, et/ou l'un au moins des intermédiaires cataboliques du MTBE (Figure 12).

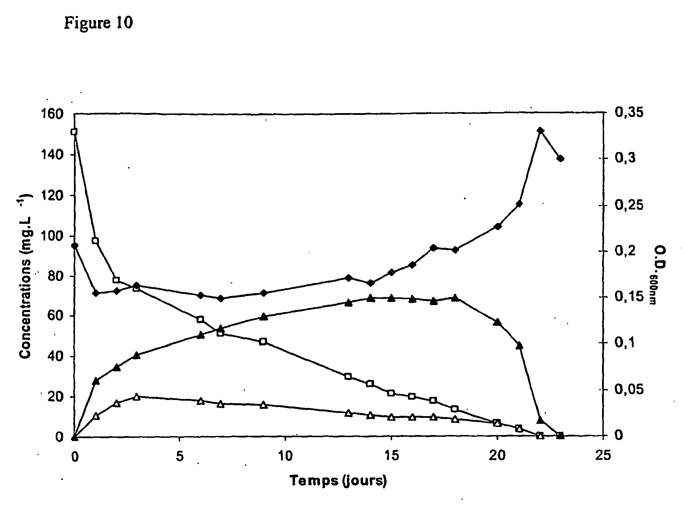

En mettant en oeuvre le procédé conforme à l'invention, une nouvelle souche bactérienne *M. austroafricanum* a été identifiée ; elle est enregistrée dans la collection CNCM sous le numéro I-3401. Elle a été isolée de l'eau de ruissellement prélevée au fond d'un réservoir de stockage d'une essence supplémentée en MTBE. La croissance de *M. austroafricanum* I-3401 sur MTBE suit une voie de dégradation similaire à celle de *M. austroafricanum* I-2562 (Figure 10). Préférentiellement, la nouvelle souche *M. austroafricanum* I-3401 est caractérisée par le fait qu'elle soit capable d'assimiler le MTBE, et/ou l'un au moins des intermédiaires cataboliques du MTBE, préférentiellement sélectionnés dans le groupe consistant en le TBA, le 2-M1,2-PD, l'hydroxyisobutyraldéhyde et le HIBA. Plus préférentiellement, la nouvelle souche I-3401 est caractérisée par une séquence nucléique de l'ADNr 16S comprenant la séquence SEQ ID NO : 13.

L'isolement indépendant de deux souches de *M. austroafricanum, I-*2562 et I-3401, toutes deux capables de croître sur MTBE et isolées de deux origines géographiques distinctes et éloignées (boues activées d'une station d'épuration d'une usine de traitement des eaux urbaines de la région parisienne et eau de ruissellement prélevée au fond d'un réservoir de stockage d'une essence supplémentée en MTBE localisée dans le sud-ouest de la France, respectivement) montre que de tels microorganismes, qui sont souvent isolés d'échantillons de sols et d'eaux (Jones et Jenkins, Can. J. Microbiol., 1965, 11 : 127-133 ; Viallier et Viallier, Ann. Soc. Belge Med. Trop., 1973, 53: 361-371), peuvent donc jouer un rôle significatif dans la dégradation du MTBE dans les aquifères contaminés.

La présente invention concerne également l'utilisation du polypeptide SEQ ID NO:2 tel que défini en a) ou b) en tant qu'aldéhyde déshydrogénase, préférentiellement pour la déshydrogénation de l'hydroxyisobutyraldéhyde en HIBA.

La présente invention concerne enfin un procédé de traitement d'effluents aqueux comprenant du MTBE, du ETBE et/ou au moins l'un des intermédiaires cataboliques du MTBE, préférentiellement sélectionné dans le groupe consistant en le TBA, le 2-M1,2-PD, l'hydroxyisobutyraldéhyde, le HIBA, afin de réduire leur concentration, comprenant l'ensemencement dudit effluent aqueux par au moins une cellule conforme à l'invention ou identifiée par le procédé d'indentification conforme à l'invention. Avantageusement, le procédé conforme à l'invention est caractérisé en ce que la cellule est fixée sur un support adapté, préférentiellement un support minéral, plus préférentiellement un support comprenant au moins 50% en masse de partite.

Le procédé selon l'invention est particulièrement adapté pour traiter un aquifère ou un sol contaminé par du MTBE, et/ou l'un au moins des intermédiaires cataboliques du MTBE.

### LISTE DES FIGURES

**Figure 1****.** Voie métabolique de dégradation du MTBE par *M. austroafricanum* I-2562. En gras, les intermédiaires de dégradation qui ont été identifiés au cours de nos expérimentations.
**Figure 2****.** Croissance de *M. austroafricanum* I-2562 sur MTBE. Pendant la croissance, la D.O. _{600 nm} (0-0) ainsi que les concentrations en MTBE (■-■), TBF(Δ-Δ) et TBA ( - ) ont été mesurées.
**Figure 3****.** Electrophorèse sur gels de polyacrylamide à 10% (A) ou 15% (B) en présence de SDS (SDS-PAGE) des extraits cellulaires de *M. austroafricanum* I-2562 obtenus après croissance sur glucose ou sur MTBE. Les poids moléculaires (en kDa) sont indiqués sur la droite de la figure.
**Figure 4****.** Amplification par PCR sur l'ADN génomique *M. austroafricanum* I-2562 avec les amorces dégénérées créées à partir des séquences peptidiques internes du polypeptide de 64 kDa induit sur MTBE.

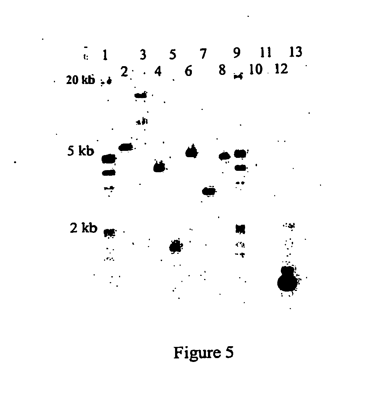

**Figure 5****.** Hybridation par Southern Blot de l'ADN génomique de *M. austroafricanum I-*2562 après digestion en utilisant la sonde de 204 pb. La taille des bandes révélées est indiquée en kb. 5 µg d'ADN génomique de *M. austroafricanum* I-2562 sont digérés par les enzymes de restriction *BamH*I*, EcoR*I*, Fsp*I*, Knp*I*, Nhe*I*, Nru, Pst*I*, Pvu* II ou *Sma*I (puits 2-8, respectivement) et 50 ng de sonde de 204 pb (puits 12) sont analysés par Southern Blot en utilisant la sonde de 204 pb. Les positions correspondant à la migration de l'ADN du marqueur de poids moléculaire III marqué avec la digoxigénine (Roche; puits 1 et puits 9) sont indiquées sur la gauche du luminographe.
**Figure 6****.** "Hybridation sur colonies" des clones recombinants d'*E. coli* DH10B avec une sonde de 604 pb.
   A) Luminographie des membranes contenant les premiers clones provenant de la digestion par *SmaI* de *M. austroafricanum* I-2562. Les clones positifs (taches noires) sont détectés avec la sonde *mpdC* marquée à la digoxigénine.
   B) Luminographie des membranes contenant les seconds clones provenant de la digestion par *Pst*I de *M. austroafricanum* I-2562. Les clones positifs (taches noires) sont détectés avec la sonde *mpdB* marquée à la digoxigénine.
**Figure 7****.** Organisation du cluster des gènes impliqués dans la voie de dégradation du MTBE et isolé à partir de l'ADN génomique de *M. austroafricanum* I-2562 (A) et leur carte de restriction correspondante (B).
   L'ORF *mpdR* code pour un régulateur transcriptionnel, les ORF *mpdC* et *mpdB* codent respectivement pour une enzyme aldéhyde déshydrogénase et une enzyme alcool déshydrogénase. Trois polypeptides de 64, 55 et 27 kDa sont tous fortement exprimés après croissance de *M. austroafricanum* I-2562 sur MTBE. Le polypeptide de 25 kDa est une perméase. Les fragments PCR issus des amplifications réalisées à l'aide des couples de primers RT-PCR-F1/-R1, RT-PCR-F2/-R2 et RT-PCR-F3/-R3 sont représentés schématiqument sur la figure 7 (fragments numérotés I, II et III).

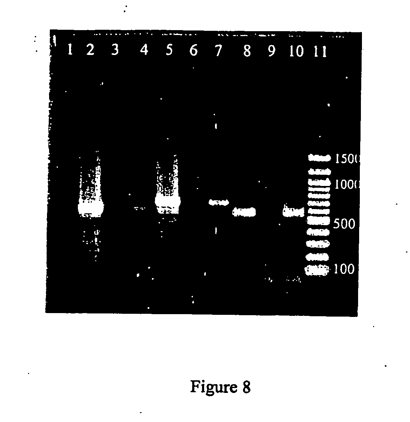

**Figure 8****.** RT-PCR sur *M. austroafricanum* I-2562 après croissance sur TBA en utilisant les amorces correspondant au cluster *mpd*.
   Les amorces utilisées sont citées dans le Tableau ci-dessous.
   Légende: Puits 1 : C⁻ PCR I, Puits 2 : C⁺ PCR I, Puits 3 :(-) RT-PCR I, Puits 4 : (+)RT-PCR I, Puits 5 :C⁺ PCR II, Puits 6 : (-) RT-PCR II, Puits 7 : (+) RT-PCR II, Puits 8 : C⁺ PCR III, Puits 9 : (-) RT-PCR III, Puits 10 : (+) RT-PCR III, Puits 11 : marqueur d'ADN (100 pb).

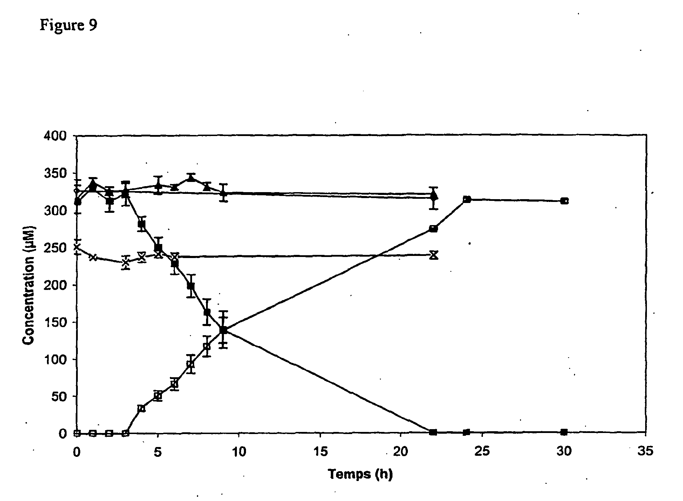

**Figure 9****.** Cinétique de dégradation du 2-M1,2-PD par des cellules de *M. smegmatis* mc2 155-clone 9 (carrés) et de *M. smegmatis* mc2 155-pCL4D (triangles).
   Dégradation du 2-M-1,2-PD (symboles pleins) et production du HIBA (symboles vides). Contrôle abiotique (0-0). Cellules de *M. smegmatis* mc2 155-clone 9 en présence de 400 mg.L⁻¹ de chloramphénicol (x-x).
   Les concentrations cellulaires de *M. smegmatis* mc2 155-clone 9 et de *M. smegmatis* mc2 155-pCL4D étaient 233,33 ± 14,09 et 214,63 ± 21,95 mg.L⁻¹, respectivement.
**Figure 10****.** Croissance de *M. austroafricanum* I-3401 sur MTBE.
   Pendant la croissance, la D. O. _{600 nm} (◆-◆), et les concentrations résiduelles en MTBE (□-□), TBF (Δ-Δ) et TBA (▲ - ▲) ont été mesurées.

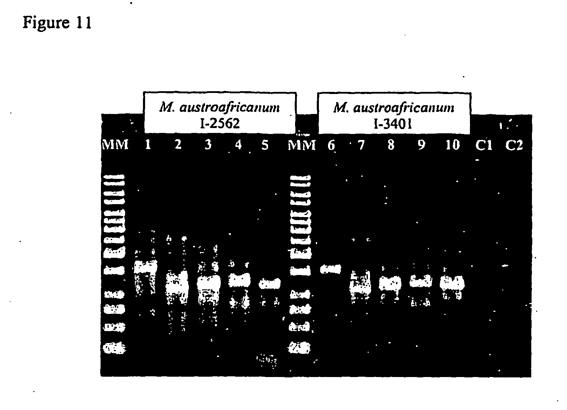

Figure 11. Amplification par PCR du cluster *mpd* dans l'ADN génomique de M. *austroafricanum* I-3401.
   Légende :
   MM : marqueur d'ADN 1kb,
   Puits 1 et 6 : amplification de SEQ ID NO:9 codant pour le polypeptide de 47 kDa.
   Puits 2 et 7 : amplification de SEQ ID NO:1 codant pour l'aldéhyde déshydrogénase de 55 kDa.
   Puits 3 et 8 : amplification de SEQ ID NO:3 codant pour la protéine de 27 kDa
   Puits 4 et 9 : amplification de SEQ ID NO:5 codant pour l'alcool déshydrogénase de 64 kDa.
   Puits 5 et 10 : amplification de SEQ ID NO:7 codant pour la protéine de 25 kDa, la perméase putative.
   C1 et C2 : contrôles négatifs sans amorces des ADN génomiques de *M. austroafricanum* I-2562 et I-3401, respectivement.
**Figure 12****.** Amplification de séquences spécifiques de l'ADNr 16S dans divers microorganismes.
   La taille attendue du produit de PCR est 331 pb.
   Les ADN génomiques des souches de *Rhodococcus ruber* (puits 1), *Rhodococcus* sp. B-1 (puits 2), *Pseudomonas resinovorans* CA10 (puits 3), *Mycobacterium smegmatis* mc2 155 (puits 4), *Escherichia coli* DH10B (puits 5), *Mycobacterium austroafricanum* I-2562 (puits 6), *Mycobacterium austroafricanum* I-3401 (puits 7) et des échantillons provenant du milieu de la cassette 1 (puits 9) et du milieu de la cassette 2 (puits 10) d'une biobarrière-pilote ensemencée avec *M. austroafricanum* I-2562 pour la dégradation du MTBE ont été utilisés pour effectuer les réactions d'amplification par PCR. GeneRuler^{™} 1kb DNA Ladder a été utilisé comme marqueur de poids moléculaire de l'ADN (puits 8).

La présente invention sera mieux comprise à l'aide des exemples qui suivent ; ces exemples sont donnés uniquement à titre d'illustration des objets de l'invention, dont ils ne constituent en aucune manière une limitation.

### EXEMPLE 1 : IDENTIFICATION DES POLYPEPTIDES INDUITS APRES CROISSANCE DE LA SOUCHE M. AUSTROAFRICANUM I-2562 SUR MTBE

Dans le but d'identifier et de détecter la présence de protéines induites spécifiquement après croissance des bactéries *M. austroafricanum* sur un milieu comprenant du MTBE, des extraits de protéines cytoplasmiques ont été réalisés à partir de culture sur MTBE ou sur glucose (Figure 2).

### 1.1. Préparation des extraits protéiques

Des cellules de la souche *M. austroafricanum* I-2562 sont cultivées sur 300 mL de milieu minéral (MM) défini précédemment décrit (Piveteau et al., Appl. Microbiol. Biotechnol. 2001, 55: 369-373) en présence de MTBE ou de glucose comme source de carbone. Les cultures sont incubées en aérobiose à 30°C dans des fioles coniques sous agitation. La croissance est évaluée par mesure de l'absorbance (D.O.₆₀₀ₙₘ) sur un spectrophotomètre UV-1601 (Shimadzu Corporation, Kyoto, Japon). Lorsque la D.O.₆₀₀ est de 1, les cellules sont collectées par centrifugation à 20,000 x g pendant 15 min, lavées deux fois dans du tampon phosphate (20 mM, pH 7) et remises en suspension dans 5 mL de tampon Tris-HCl à 50mM (pH=8,0) contenant 0,1M de dithiothreithol (DTT). Les cellules sont cassées par trois passages à la presse de French (20,000 psi) et toujours maintenues dans la glace. Les débris cellulaires sont éliminés par deux centrifugations à 1,000 x g pendant 2 min. Le surnageant est alors utilisé pour l'analyse du profil protéique. La concentration en protéines totales est mesurée par le kit Bio-Rad (Dye reagent, Bio-Rad laboratories GmbH, Munich, Allemagne).

### 1.2. Analyse des protéines solubles

L'électrophorèse sur ces extraits est effectuée sur des gels SDS-PAGE à 10 ou 15% de polyacrylamide contenant du SDS selon la méthode mise au point par Laemmli mis à part pour les conditions de migration (V =150 Volts pendant la première heure puis A=33mA de façon constante pendant les heures suivantes de la migration). 12,5 µg de protéines totales sont déposées par puits sur le gel, pour chaque source de carbone testée (Laemmli, Nature, 1970, 227: 680-685).

Les profils protéiques de haut et de bas poids moléculaires révélés sur gels SDS-PAGE à 10 ou 15% d'acrylamide, respectivement, ont permis de détecter 9 protéines de poids moléculaires différents spécialement induites par la présence du MTBE (Figure 3).

### 1.3. Identification de deux séquences peptidiques du polypeptide MpdC (64 kDa) induit durant la croissance sur MTBE

### Protocole du séquençage interne:

Chaque bande de gel correspondant à un polypeptide induit spécifiquement sur le profil protéique après croissance sur MTBE de *M. austroafricanum* I-2562 est découpée puis soumise à une réduction au DTT "dans le gel" puis à une alkylation des ponts disulfure par l'iodoacétamide (Jenö et al. Analytical Biochemistry. 1995, 224: 75-82) avant digestion à la trypsine. L'enzyme utilisée est la trypsine de qualité "sequencing grade" de Promega (Helman et al. 1995, William et al., In: Techniques VIII, Marshak, D., ed. Academic Press, San Diego, 1997, 79-90). Les peptides ainsi générés sont alors extraits des morceaux de gel résiduels par 3 traitements de 30 min à 60°C, chacun en présence de 100 µL de TFA à 1% et acétonitrile à 60% suivi d'un passage au sonicateur pendant 10 min. Une dernière extraction est effectuée dans 50 µL d'acétonitrile pur pendant 10 min. Les 4 extraits sont mélangés et le volume liquide est réduit dans un évaporateur rotatif (Savant AES 1010) pour obtenir un volume final de 5µL.

Les peptides sont alors séparés par HPLC (Vydac) sur une colonne microbore C18 (300°A ; 1 X 50mm #218TP5105) en utilisant le système de séparation Applied Biosystems 130A. Les peptides sont élués à 100 µL/min par un mélange de solvants A et B avec un gradient de mélange des solvants programmé ainsi: 3-63 min (0-50% B), 63-72min (50-100% B) et 72-75min (100% B). Le solvant A est constitué de TFA à 0,1% dans l'eau et le solvant B est composé de TFA à 0,08 % dans 70% d' acétonitrile et eau, les peptides sont détectés par un détecteur UV à 220 nm. Les différentes fractions sont collectées manuellement dans des tubes de 1,5 mL et déposées sur des disques en fibre de verre prétraités par addition de 7 µL de biobrène (Applied Biosystems Inc.). Les disques sont soumis à la dégradation d'Edman sur un séquenceur Procise (modèle 494 cLC) selon le protocole décrit par Hewick et al. (J. Biol. Chem., 1981, 256:7990-7997).

Une quantité de peptide équivalente à environ 1 pmole est déposée sur le séquenceur et un programme standard utilisant du TFA comme phase liquide est utilisé pour le séquençage. Les dérivés acides aminés-Phénylthiohydantoine (PTH-aa) sont déterminés par comparaison avec des standards (PTH-standards, ABI) et analysés en ligne grâce à un système de séparation capillaire (ABI 140D) dès le démarrage du séquençage.

### Résultats :

Parmi les protéines identifiées, une protéine migrant à 64 kDa (dénommée MpdC) est la plus fortement induite. L'extraction à partir de la bande de gel correspondante, la digestion trypsique suivie de micro-séquençages peptidiques internes ont permis l'obtention de 2 séquences de ce polypeptide.

La comparaison à l'aide du logiciel BLAST de la première séquence interne (**KQRGWAYDPNVRGLPE**) n'a pas révélé de similitudes significatives. En revanche l'analyse BLAST de la deuxième séquence interne (**STEHGLEGTIDWPISYEELAPYYDENDAIY**) montre une forte similitude (93%) avec de nombreuses oxydases et déshydrogénases appartenant à la famille des GMC-oxydoréductases (Cavener, J. Mol. Biol., 1992, 223 : 811-814).

### EXEMPLE 2 : PREPARATION DES SONDES

### 2.1. Extraction de l'ADN génomique

L'ADN génomique a été isolé des différentes souches testées selon le protocole de Pospiech et Neumann modifié de la façon suivante : une culture effectuée sur 50 mL de milieu LB (D.O. à 600nm de 0,7) est collectée par centrifugation à 10000 g pendant 15min à 4°C (Pospiech and Neumann, Trends In Genetics., 1995, 11: 217-218). Le culot est remis en suspension dans 5 mL de tampon SET (NaCl 75mM, EDTA 25mM, Tris-HCl 20mM à pH8). Du lysozyme est ajouté à une concentration finale de 1 mg.mL⁻¹ ainsi que de la lysostaphine à 112 U.µL⁻¹ (Sigma). Ce mélange réactionnel est incubé pendant 1 h à 37°C. Après traitement par le mélange SDS/Protéinase K, une étape d'extraction est effectuée dans le mélange réactionnel suivant où les différents réactifs sont ajoutés séquentiellement : ¼ volume de NaCl 5M suivi de 1/7,5 volume de CTAB à 10% - NaCl 0,7M (pré-chauffé à 65°C) et le mélange est incubé pendant 1h à 65°C. Un volume de chloroforme (CHCl₃) est ensuite ajouté et les étapes subséquentes sont réalisées selon le protocole original.

### 2.2. Préparation d'une sonde de 204 pb

Les deux séquences peptidiques obtenues dans l'exemple 1 ont été utilisées pour créer plusieurs paires d'amorces dégénérées en accord avec le code génétique universel et en tenant compte des deux possibilités d'appariement des séquences au sein de la séquence polypeptidique. La connaissance de ces séquences peptidiques permet, par le jeu du code génétique, de déduire les séquences nucléotidiques correspondantes. Le code génétique équivaut à la correspondance entre les séquences nucléotidiques et les séquences peptidiques. Ainsi, chaque triplet de nucléotides de la séquence nucléotidique ou codon correspond à un acide aminé unique. On parle de dégénérescence du code génétique dans la mesure où plusieurs codons synonymes peuvent spécifier un même acide aminé. En conséquence, par le jeu du code génétique, plusieurs séquences nucléotidiques peuvent être déduites pour une séquence peptidique unique. A partir de ces informations, on conçoit et synthétise des amorces oligonucléotidiques dégénérées : elles servent d'amorces dans le cadre d'une réaction d'amplification à l'issue de laquelle on obtient une sonde ayant une grande spécificité pour le ou les acides nucléiques recherchés.

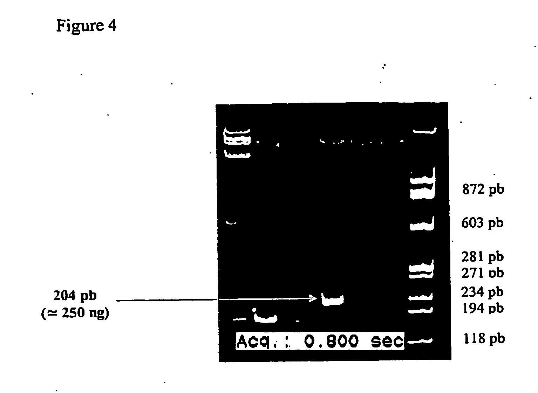

Un seul couple d'amorces (MadF1/MadR1) a permis l'obtention par PCR, à partir d'ADN génomique pure de *M. austroafricanum* I-2562, d'un fragment d'acide nucléique de 204 pb (figure 4).
La séquence nucléotidique de l'amorce MadF1 est :
5'- GGNTGGGCNTAYGAYCC-3'
La séquence nucléotidique de l'amorce MadR1 est :
5'- GCRTCRTTYTCRTCSTAST-3'

La séquence en acides aminés a été obtenue grâce au logiciel ORF finder (http://www.ncbi.nlm.nih.gov/gorf/gorf.html). Elle est représentée par :

L'analyse BLAST de cette séquence en acides aminés confirme l'appartenance de la protéine MpdC à la famille des GMC-oxydoréductases puisque l'analyse montre 62 % de similitude (44% d'identité sur 45 acides aminés) avec une choline déshydrogénase putative de *Bradyrhizobium japonicum,* 53 % de similitude (38% d'identité sur 76 acides aminés) avec une glucose déshydrogénase d'une souche de *Burkholderia cepacia* et 61 % de similitude (38% d'identité sur 47 acides aminés) avec une oxydoréductase, toutes appartenant à cette même famille.

### 2.3. Préparation d'une sonde de 604 pb

A la suite des informations obtenues à partir de cette première amplification PCR, des amorces dégénérées ont été conçues et synthétisées en tenant compte de certains motifs conservés qui sont présents dans les protéines de la famille des GMC-oxydoréductases. Un des couples d'amorces (MadF2/MadR2) a permis d'obtenir un nouvel acide nucléique de 604 pb.
La séquence nucléotidique de l'amorce MadF2 est :
5'- TTCACCTTGTTGGAACCGCTGGG-3'
La séquence nucléotidique de l'amorce MadR2 est :
5'- TCATTACCGAGCCGACCTGC-3'

Les sondes sont marquées à la dUTP-digoxigenine (DIG DNA Labelling and Detection Kit; Roche Diagnostics, Laval Canada).

### EXEMPLE 3: CLONAGE D'UN ACIDE NUCLEIQUE SEQ ID NO:11

### 3.1. Southern blot

Les expériences de Southern Blot ont été réalisées sur de l'ADN génomique de la souche *M. austroafricanum* I-2562 tel que préparé dans l'exemple 1. 5 µg d'ADN génomique est partiellement digéré à l'aide d'enzymes de restriction choisies en fonction de leur site de restriction *BamH*I, *EcoR*I, *Fsp*I, *Knp*I, *Nhe*I, *Nru, Pst*I, *Pvu* II ou SmaI (puits 2-8, respectivement sur la figure 5). Après migration sur gel, les ADN digérés sont transférés sur une membrane de nylon. A l'aide de la sonde de 204 pb marquée à la dUTP-digoxigenine (DIG DNA Labelling and Detection Kit; Roche Diagnostics, Laval Canada), on procède au criblage par hybridation des acides nucléiques digérés. La sonde de 204 pb s'hybride spécifiquement à des fragments d'acides nucléiques compris entre 2 et 12 kb (figure 5). Les positions correspondant à la migration de l'ADN du marqueur de poids moléculaire III marqué avec la digoxigénine (Roche; puits 1 et puits 9) sont indiqués sur la gauche du luminographe.

### 3.2. Construction de mini-banques, criblage et clonage

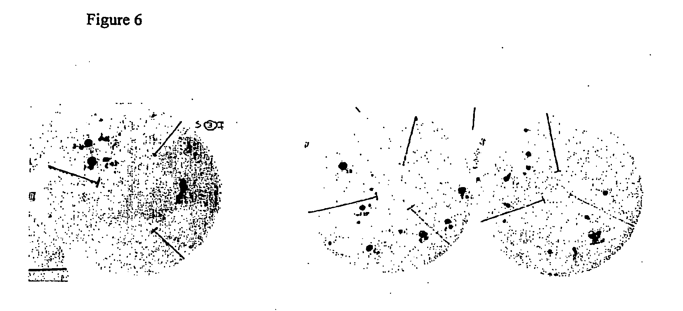

Ces fragments d'acides nucléiques sont clonés dans le plasmide pBluescript II KS (pBKS) (+/-) après digestion par *Sma*I. Les plasmides recombinants sont utilisés pour transformer des bactéries compétentes DH10B d'*E*. *coli* préparées (Hanahan, D et al., Methods Enzymol. 1991, 204:63-113). Chaque colonie bactérienne issue d'un clone bactérien renfermant un plasmide recombinant est transférée sur une membrane de nitrocellulose et criblée par hybridation sur colonie avec la sonde de 604 pb telle qu'obtenue dans l'exemple 2.3. Pour chaque clone positif, c'est-à-dire s'hybridant avec la sonde, le plasmide recombinant (pKS1) est extrait et purifié grâce au kit QIAprep Spin Miniprep (QIAGEN, Mississauga, Canada). Etant donné la forte instabilité du fragment *Sma*I au sein du plasmide pBKS, entraînant l'apparition de plasmides de taille variées, une digestion par *Sma*I de 10 µg de plasmide pBKS1 suivie d'une extraction sur gel a été réalisée. L'extraction de la bande *Sma*I a été obtenue grâce à une extraction après migration sur un gel « low melting point ». Environ 600 à 1000 paires de base ont été obtenues pour chaque extraction, par conséquent une douzaine d'extraction ont été nécessaires pour obtenir une séquence complète. Après analyse du séquençage, la partie 5' en amont du gène *mpdC* étant manquante, une nouvelle mini-banque a été réalisée afin d'obtenir un fragment *Pst*I de 5,6 kb en utilisant les même techniques que précédemment, mais en utilisant la sonde de 914 pb chevauchant le premier insert et obtenue à l'aide du couple d'amorces MF3/MR3. Le plasmide pKS3 a été isolé à partir d'un clone positif révélé par « hybridation sur colonies » (Figure 6). Le fragment *Pst*I*-Pst*I détecté par la sonde a donc été cloné selon le même protocole que précédemment afin d'obtenir un plasmide recombinant pKS3.

L'intégralité de l'insert PstI présent dans ce plasmide a été séquencée selon la technique de « primer-walking » à l'aide d'une T7 ADN polymérase (T7-DNA sequencing kit, Applied Biosystems, Foster City, CA) et d'un séquenceur (ABT prism 377 automated fluorescence sequencer ; Applied Biosystems, Foster City, CA). Les séquences nucléotidiques et d'acides aminées correspondantes ont été comparées avec celles contenues dans les bases de données EMBL, Swissprot et GenBank en utilisant les logiciels BLASTN et BLASTX du National Center for Biotechnology Information (NCBI).

### EXEMPLE 4: IDENTIFICATION ET CARACTERISATION DES DIFFERENTS CADRES OUVERTS DE LECTURE (ORF) COMPRIS DANS LA SEQUENCE SEQ ID NO: 11 DE M. AUSTROAFRICANUM I-2562

L'analyse des cadres ouverts de lecture présents dans la séquence d'ADN *Pst*I/*Sma*I, SEQ ID NO:11 obtenue à l'issue des deux clonages décrits dans l'exemple 3 a été réalisée à l'aide du logiciel « ORF finder ». Cinq séquences codantes ont été identifiées SEQ ID NO1, SEQ ID NO:3, SEQ ID NO :5, SEQ ID NO :7 et SEQ ID NO :9 ; elles codent respectivement pour les polypeptides SEQ ID NO : 2, SEQ ID NO :4, SEQ ID NO :6, SEQ ID NO :8 et SEQ ID NO :10 (cf tableau ci-dessous).

| Séquence nucléotidique | ORF | Séquence en acides aminés du polypeptide | Nom du polypetide | Poids moléculaire |
|---|---|---|---|---|
| SEQ ID NO :1 1515 pb | *mpdC* | SEQ ID NO :2 552 acides aminés | MpdC | 55 kDa |
| SEQ ID NO : 3 648 pb | *orf1* | SEQ ID NO :4 215 acides aminés | | 27 kDa |
| SEQ ID NO : 5 1659 pb | *mpdB* | SEQ ID NO :6 552 acides aminés | MpdB | 64 kDa |
| SEQ ID NO :7 672 pb | *orf2* | SEQ ID NO :8 223 acides aminés | | 25 kDa |
| SEQ ID NO :9 1233 pb | *mpdR* | SEQ ID NO :10 410 acides aminés | MpdR | 47 kDa |

La séquence en acides aminés SEQ ID NO :6 du polypeptide MpdB de 64 kDa a donc pu être corrélée avec les premières séquences polypeptidiques et les amplifications PCR de 204 et 604 pb. Ce gène *mpdB* est compris dans une organisation génétique comprenant 3 autres ORFs dans la même orientation, *mpdC, orf1* et *orf2,* codant respectivement pour les protéines de 55, 27 et 25 kDa. En amont de ce cluster, un nouveau gène orienté dans le sens opposé au premier cluster et codant pour une protéine de 47 kDa a été identifié et nommé *mpdR.* L'organisation de ce système est schématisée dans la Figure 7.

### 4.1. ORF mpdC, SEO ID NO :1

L'analyse par le logiciel BLAST schématisée ci-dessous montre de fortes similitudes avec de nombreuses déshydrogénases appartenant à la famille des aldéhydes déshydrogénases. Le polypeptide SEQ ID NO:2 codé par cet ORF correspond à un polypeptide de 55 kDa, dont l'expression est induite sur MTBE. Ce polypeptide est capable de déshydrogéner l'hydroxybutyraldéhyde en HIBA. 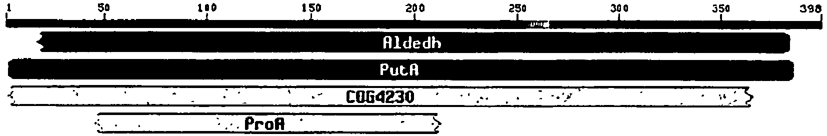 Résultat obtenu par le logiciel "NCBI domain search" :

| | |
|---|---|
| **Aldedh**, Aldehyde dehydrogenase family | e-value = 3e-114 |
| **PutA**, NAD-dependent aldehyde dehydrogenases | e value = 5e-106 |
| **COG4230**, Delta 1-pyrroline-5-carboxylate dehydrogenase | e value = 3e-44 |

### 4.2. ORF orf1, SEQ ID NO :3

Le polypeptide SEQ ID NO :4 codé par cet ORF correspond à un polypeptide de 27 kDa, dont l'expression est induite sur MTBE.

### 4.3. ORF mpdB, SEQ ID NO :5

Le polypeptide SEQ ID NO :6 codé par cet ORF correspond à un polypeptide de 64 kDa, dont l'expression est induite sur MTBE. Il a de fortes similitudes avec de nombreuses oxydases et déshydrogénases appartenant à la famille des GMC (Glucose-Méthanol-Choline) oxydoréductases. L'analyse par le logiciel "NCBI Conserved Domain Search" a détecté plus précisément des similitudes avec la protéine codée par le gène *betA,* codant pour la choline déshydrogénase appartenant à cette même famille. La séquence en acides aminés déduite peut être corrélée avec la séquence N-terminale et les deux séquences internes de la protéine de 64 kDa induite spécifiquement sur MTBE et responsable de la transformation du 2-M1,2-PD en hydroxybutyraldéhyde. 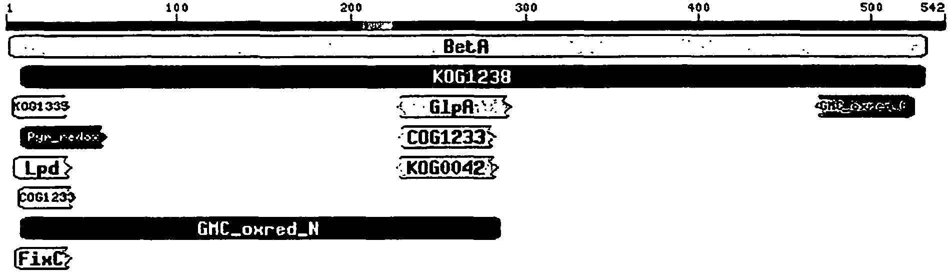 NCBI conserved domain search result
Résultat obtenu par une recherche des domaines conservés par NCBI

| | |
|---|---|
| **BetA**, Choline dehydrogenase and related flavoproteins | e-value = 3e-48 |
| **KOG1238**, Glucose dehydrogenase/choline dehydrogenase | e-value = 2e-07 |
| **KOG1335**, Dihydrolipoamide dehydrogenase [Energy production]... | e-value = 6e-05 |
| **GMC_oxred_N, GMC** oxidoreductase | e-value = 2e-03 |
| **GMC_oxred_C, GMC** oxidoreductase | e-value = 4e-03 |

### 4.4. ORF orf2, SEQ ID NO :7

Le polypeptide SEQ ID NO :8 codé par cet ORF correspond à un polypeptide de 25 kDa. L'analyse BLAST a révélé une homologie avec une Di/Tripeptide perméase (COG3104: Dipeptide/tripeptide permease [*Rubrivivax gelatinosus* **PM1**] Length = 372, Expect = 2e-24). L'analyse de l'hydropathie de cette protéine par le logiciel "Predict Protein" a révélé la présence de 5 segments transmembranaires dont l'organisation est schématisée ci-dessous :
L'expression de ce polypeptide est probablement induite en présence de MTBE.
Cette protéine se trouve donc au sein de la membrane des bactéries de l'espèce *M*. *austroafricanum.*

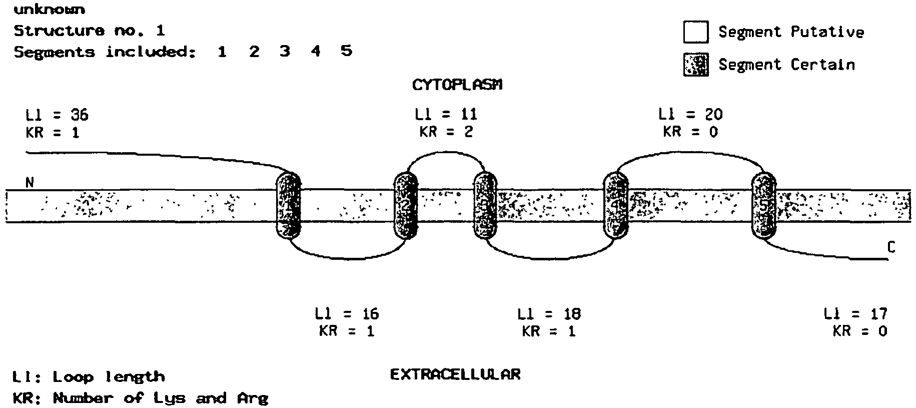

### 4.5. ORF mpdR, SEO ID NO :9

Le polypeptide SEQ ID NO :10 codé par cet ORF correspond à un polypeptide de 47 kDa. Cet ORF est situé en amont des quatre ORF précédentes et est orienté dans l'autre sens comme le montre la Figure 7. L'analyse BLAST révèle une similitude de la première moitié de la séquence avec de nombreux régulateurs transcriptionnels σ54, dont notamment de type AcoR :

| | |
|---|---|
| **Sigma-54 dependent transcriptional activator** [*Azoarcus* sp. EbN1] | e = 6e-23 |
| **COG3284:** AcoR Transcriptional activator of acetoin/glycerol metabolism | e = le-22 |

D'après ces analyses, il n'est pas possible de détecter si l'expression de ce polypeptide est induite spécifiquement ou pas sur le profil MTBE mais il est possible de détecter son expression lors d'analyse RT-PCR. Elle joue potentiellement un rôle dans la régulation de l'expression des gènes *mpd.*

### EXEMPLE 5 : EXPRESSION PAR RT-PCR DU CLUSTER DES GENES mpd

### 5.1. Extraction de l'ARNm de M.austroafricanum I-2562

Pour les expériences d'extraction d'ARN, tous les ustensiles, toutes les solutions et la vaisselle ont été préparés de manière à éviter la contamination par des ARNases selon les procédures standards (Sambrook et al., Molecular Cloning: A Laboratory Manual. Cold Spring Harbor Laboratory Press, Cold Spring Harbor, New York. 2001).

L'ARN total de *M. austroafricanum* I-2562 a été extrait à partir de 20mL d'une culture à O.D.₆₀₀ₙₘ de 0,7. Après une incubation sur glace de 30 min, les cellules ont été centrifugées (8000 xg pendant 10min à 4°C) et conservées sur glace. Par la suite, 600µL de lysozyme (Roche Diagnostics, Laval, Canada) à 3mg.mL⁻¹ et 600µL de lysostaphine (Sigma, St-Louis, Missouri) à 50µg.mL⁻¹ ont été ajoutés au culot de cellules et le mélange est incubé à 37°C durant 10 min. 10mL de RNAwiz^{™} (Ambion, Austin, Texas) ont été par la suite ajoutés et le mélange est vortexé pendant 15 sec. Le mélange est ensuite fractionné dans des tubes de 2mL (1400 µL/tube) contenant 250 mg de billes de zirconium-silica (0,1 mm de diamètre) placés dans un adaptateur pour vortex (Ambion) et vortexés pendant 10 min. Après avoir centrifugé (13000 xg à 4°C pendant 5 min), le lysat bactérien correspondant aux surnageants de chaque tube est transféré dans de nouveaux tubes de 2 mL et 0,2 volume de CHCl₃ est ajouté dans chaque tube. Les tubes sont ensuite vortexés pendant 30 sec, incubés à température ambiante pendant 10 min, puis centrifugés (13000 xg à 4°C pendant 5 min) afin de récupérer les surnageants dans de nouveaux tubes de 2mL. Les extraits d'ARN sont précipités en ajoutant séquentiellement 0,5 volume d'H₂O traitée avec du DEPC, 1 volume d'isopropanol et 1/50^{ème} de volume de glycogène Rnase-free à 5 mg.mL⁻¹ (Ambion), bien mélangés et incubés à température ambiante pendant 10 min. L'ARN est centrifugé (13000 xg à 4°C pendant 5min), lavé deux fois avec de l'éthanol à 70%, séché 30 sec (DNA Speed Vac, Savant) et resuspendu dans l'H₂O-DEPC (25µL/tube). La qualité de l'ARN a été déterminé par électrophorèse à l'aide d'un gel d'agarose à 1% non dénaturant dans un tampon TBE 0,5X (Trizma Base 45mM, acide borique 45mM, Na₂EDTAlmM à pH=8,3). L'ARN est précipité avec 1/10^{ème} volume d'acétate de sodium 3M à pH=7,0 et 2,5 volume d'éthanol à 95%, puis est conservé à -80°C.

### 5.2. Traitement à la DNase et Reverse Transcriptase-PCR (RT-PCR)

Deux mg d'ARN extraits de *M. austroafricanum* I-2562 ont été traités avec le kit « DNA-Free » (Ambion, Austin, Texas) selon les recommandations du fabricant. L'élimination de l'ADN a été vérifiée à l'aide d'amplification PCR en utilisant les amorces spécifiques créées dans le cluster *mpd* et par électrophorèse sur gel d'agarose à 1%. La concentration finale d'ARN a été quantifiée à 260 nm à l'aide d'un Nanodrop (Nanodrop Technologies, Wilmington, USA). Les expériences de Reverse Transcriptase-PCR (RT-PCR) ont été mises en place selon le protocole du kit « One step RT-PCR » de QIAGEN (QIAGEN, Mississauga, Canada) tel que décrit par le fabricant. On a utilisé 120 ng d'ARN à chaque tentative de RT-PCR avec, à chaque fois en parallèle, une amplification PCR contrôle positive (et négative) avec (ou sans) 100 ng d'ADNg de *M. austroafricanum* I-2562. Les conditions de RT-PCR sont décrites ci-dessous.
- étape de reverse transcription :
   1) 30 min à 50°C
   2) ∞ à 50°C
- étape PCR:
   15 min à 95°C
   30 à 40 cycles (variables en fonction de l'intensité du signal) :
      1) 30 sec à 94°C
      2) 1 min à 55°C
      3) 1 min à 72°C
- étape d'élongation finale:
   1) 10min à 72°C
   2) ∞ à 4°C

### 5.3. Expression par RT-PCR du cluster des gènes mpd pendant la croissance de M.austroafricanum I-2562 sur TBA

L'expression des gènes *mpd* est testée après croissance des bactéries sur TBA, le principal intermédiaire de la biodégradation du MTBE (voir Figure 1). Des paires d'amorces chevauchant les différents gènes *mpd* sont créées (voir Tableau ci-dessous) afin de vérifier que l'ARNm correspondant à ce cluster était transcrit en un monocistron. L'ARN total est extrait des cultures après croissance sur TBA et les RT-PCR sont réalisées avec les différentes amorces. Le résultat de cette expérimentation qui est présentée dans la Figure 8 montre que très probablement la transcription du cluster des gènes *mpd* est monocistronique ; les gènes étant organisés en opéron. (Ma et al. J. Bacteriol., 2002, 184 : 5733-5745).

**Tableau : Amorces utilisées dans l'ensemble des expériences d'amplification par PCR et RT-PCR :**

| Amorces | Séquence | Région amplifiée |
|---|---|---|
| Bott1 (SEQ ID N :15) | 5'-TGCACACAGGCCACAACCCA-3' | ADNr 16S |
| Bott2 (SEQ ID N :16) | 5'-GAGAGTTTGATCCTGGCTCAG-3' | ADNr 16S |
| 244 (SEQ ID N : 17) | 5'-CCCACTGCTGCCTCCCGTAG-3' | ADNr 16S |
| Tb11 (SEQ ID N : 18) | 5'-ACCAACGATGGTGTGTCCAT-3' | *hsp65* |
| Tb12 (SEQ ID N :19) | 5'-CTTGTCGAACCGCATACCCT-3' | *hsp65* |
| MaFV2 (SEQ ID N :20) | 5'-GTCTAATACCGAATACACCCTTCT-3' | ADNr 16S |
| MaRV6 (SEQ ID N :21) | 5'-GTAGTTGGCCGGTCCTTCTTCTCC-3' | ADNr 16S |
| MF1 (SEQ ID N:22) | 5'-TGAGAAGCCTCGTGTATTAC-3' | *orf1-mpdB* |
| MR1 (SEQ ID N :23) | 5'-GAGATAAGGCGTGGTGAA-3' | *orf1-mpdB* |
| MF2 (SEQ ID N :24) | 5'-AGTGACGGCACCCATAAGTG-3' | *mpdR* interne |
| MR2 (SEQ ID N :25) | 5'-TCGAGGTGTTGAGGTCCGAAT-3' | *mpdR* interne |
| MF3 (SEQ ID N :26) | 5'-ATCATCCCGTGGAACTAC-3' | *mpdC* interne |
| MR3(SEQ ID N :27) | 5'-TGACCTGGGCGATGTGTT-3' | *mpdC* interne |
| MF4 (SEQ ID N :28) | 5'-ATCAGACCTGGGATGTGC-3' | *mpdB-orf2* |
| MR4 (SEQ ID N :29) | 5'-GGCTGTGAAAGTCGGATGA-3' | *mpdB-orf2* |
| | | |
| RT-PCR-F1 (SEQ ID N :30) | 5'-AGTGACGGCACCCATAAGTG-3' | *mpdR* interne |
| RT-PCR-R1 (SEQ ID N :31) | 5'-TCGAGGTGTTGAGGTCCGAAT-3' | *mpdR* interne |
| RT-PCR-F2 (SEQ ID N :32) | 5'-GCAGGTCGGCTCGGTAATGA-3' | *mpdC-orf1* |
| RT-PCR-R2 (SEQ ID N :33) | 5'-GTAATACACGAGGCTTCTCA-3' | *mpdC-orf1* |
| RT-PCR-F3 (SEQ ID N :34) | 5'-ACGGTCTCGTCGGCAAATAC-3' | *mpdB-orf2* |
| RT-PCR-R3 (SEQ ID N :35) | 5'-GCACATCCCAGGTCTGAT-3' | *mpdB-orf2* |

### EXEMPLE 6: EXPRESSION DES GENES mpd DANS LA BACTERIE DE LA SOUCHE M. SMEGMATIS MC2 155

### 6.1. Construction du vecteur d'expression contenant les gènes mpd et transformation des bactéries

Les gènes compris dans l'organisation génétique *mpd* ont été insérés au sein du vecteur pCL4D en deux étapes (Picardeau et al., Microbiology, 2000, 146: 305-313). Dans un premier temps, le fragment *Not*I*-Not*I de 4401 paires de bases contenu dans le plasmide pKS 1 a été digéré par *Not*I (NEB, Pickering, Canada) et cloné dans le site *Hinc*II de pCL4D. Les cellules chimiocompétentes d'*E*. *coli* DH10B ont été utilisées pour la transformation et la sélection des transformants sur du milieu LB contenant 20 µg.mL⁻¹ de kanamycine. La détection des clones positifs contenant le plasmide recombinant p4D1 s'est faite par amplification PCR à l'aide des amorces Forward et Reverse (Tableau dans l'Exemple 5). Lors de la seconde étape, le fragment *Pst*I de 5574 pb contenant la partie manquante du gène *mpdC,* le gène *mpdR* et l*'orf3* a été extrait par digestion du plasmide pKS3 à l'aide de l'enzyme *Pst*I. Ce fragment a été introduit au sein du plasmide p4D1 digéré au préalable par *Pst*I permettant ainsi d'enlever le fragment PstI correspondant à la sonde de 914 pb présent dans le plasmide p4D1. L'orientation du fragment PstI dans le plasmide recombinant p4D2 contenant la totalité du cluster *mpd* a été vérifiée par PCR.

Dans le but d'exprimer l'ensemble des gènes clonés, le vecteur p4D2 a été introduit dans des cellules compétentes de la souche *M. smegmatis* mc2 155 en utilisant la technique d'électroporation. Un transformant (souche *M. smegmatis* mc2 155-clone 9) issu de cette transformation a été isolé sur boîte LB contenant de la kanamycine (20 µg.mL⁻¹). Le plasmide pCL4D ne comprenant pas d'insert a également été introduit dans des bactéries de la souche *M. smegmatis* mc2 155 (souche *M*. *smegmatis* mc2 155-pCL4D) et sert de contrôle.

### 6.2. Expression fonctionnelle des gènes mpdB et mpdC chez M. smegmatis mc2 155

Les bactéries *M. smegmatis* mc2 155-clone 9 (avec insert) et *M. smegmatis* mc2 155-pCL4D (contrôle) sont conservées sur du milieu LB contenant 20 mg.L⁻¹ de kanamycine. Les deux souches sont cultivées sur 200 mL de milieu LB contenant 20 mg.L⁻¹ de kanamycine pendant 72 h à 30°C. Les cellules de *M*. *smegmatis* mc2 155-clone 9 et *M. smegmatis* mc2 155-pCL4D sont collectées par centrifugation (13,000 g pendant 15 min), lavées deux fois et mises en suspension dans 40 mL de tampon phosphate (20 mM, pH 7) contenant le substrat à tester (MTBE, TBA, 2-M-1,2-PD ou HIBA) dans des fioles scellées de 120 mL. Après ensemencement, les fioles sont incubées à 37°C sur un agitateur rotatif. Lorsque c'est nécessaire, du chloramphénicol est ajouté à partir d'une solution dans l'eau stérilisée par filtration (0,22 pm) afin d'obtenir une concentration finale de 400 mg.L⁻¹. Les échantillons filtrés de ces cultures sont analysés par CPG ou HPLC. La dégradation du substrat est suivie sur une période de 24 h. Les activités spécifiques (mg de substrat dégradé. g⁻¹ de biomasse.h⁻¹) sont calculées à partir des vitesses maximales de dégradation.

### 6.3. Expression chez M. smegmatis mc2 155 du cluster des gènes mpd isolés de M. austroafricanum 1-2562

Un fragment de 9,1 kb, SEQ ID NO :11 contenant le cluster des gènes *mpd* de *M. austroafricanum* I-2562 a été cloné dans le plasmide pCL4D (Picardeau et al. 2000). Ce plasmide, nommé p4D2, a ensuite été utilisé pour effectuer la transformation de *M. smegmatis* mc2 155. La transformation a été réalisée aussi avec le plasmide pCL4D, comme contrôle. Deux transformants ont été sélectionnés : *M*. *smegmatis* mc2 155-clone 9 (contenant p4D2 et portant donc les gènes *mpd*) et *M*. *smegmatis* mc2 155-pCL4D (portant le vecteur, pCL4D). Les deux souches sont cultivées sur du milieu LB contenant de la kanamycine puis testées dans des expériences en resting cells pour la capacité à dégrader le MTBE, le TBA, le 2-M1,2-PD et le HIBA. Aucune dégradation du MTBE, du TBA ou du HIBA n'a été observée avec les souches *M. smegmatis* mc2 155-clone 9 et *M. smegmatis* mc2 155-pCL4D (Tableau 2).

La dégradation du 2-M 1,2-PD et la production stoechiométrique de HIBA (312,1 ± 2,3 µM dégradé et 311,6 ± 4,5 µM produit, respectivement) a été observé seulement en présence de la souche *M. smegmatis* mc2 155-clone 9 (Figure 9). Aucune dégradation du 2-M 1,2-PD n'a été observée en présence de la souche *M*. *smegmatis* mc2 155-pCL4D ou dans le contrôle abiotique. Les deux souches étant cultivées sur milieu complet LB, l'induction des gènes était nécessaire pour produire les enzymes correspondantes et c'est pourquoi la dégradation ne commence qu'après 4 h d'incubation en présence de 2-M1,2-PD Aucune dégradation du 2-M1,2-PD n'est observée quand les cellules de *M. smegmatis* mc2 155-clone 9 sont incubées en présence de 2-M1,2-PD et de chloramphénicol qui est connu pour inhiber la traduction des ARNm en protéines.

La vitesse maximale de dégradation du 2-M-1,2-PD par la souche n *M. smegmatis* mc2 155-clone 9 a été calculée et est de 2,34 ± 0.41 µmol.g⁻¹ (poids sec).min⁻¹.

### EXEMPLE 7 : IDENTIFICATION D'UNE NOUVELLE SOUCHE M. AUSTROAFRICANUM I-3401, CAPABLE DE CROITRE SUR MTBE

L'eau de ruissellement collectée au fond d'un réservoir de stockage d'une essence supplémentée en MTBE a été utilisée pour ensemencer du milieu minéral MM contenant 200 mg.L⁻¹ de MTBE. Le MTBE a été lentement mais totalement consommé en 140 jours. Toutes les bactéries formant des colonies après étalement sur boîtes de milieu LB plates ont été individuellement réisolées puis testées pour leur capacité à pousser sur MM contenant du MTBE comme seule source de carbone et d'énergie. Une bactérie Gram positive et aérobie stricte formant des bâtonnets, et poussant sous forme de colonies jaunes sur boîtes de milieu LB a été isolée et s'est avérée capable de croissance sur MTBE.

L'ADNr 16S de la souche I-3401 a été totalement séquencé pour les deux brins et le gène *hsp65* a été partiellement séquencé. La séquence de l'ADNr 16S montre que la nouvelle souche I-3401, SEQ ID NO:13, est très proche de *M*. *austroafricanum* I-2562, avec sept nucléotides différents (5 substitutions et 2 insertions chez I-3401). La séquence du gène *hsp65* diffère par huit nucléotides de celle du gène *hsp65* de la souche-type (de référence) de *M. austroafricanum.* La capacité de *M. austroafricanum* I-3401 à pousser sur MTBE a été montrée (Figure 10).

### EXEMPLE 8: CREATION D'AMORCES SPECIFIQUES DE L'ADNr 16S DE M. AUSTROAFRICANUM ET CONDITIONS SPECIFIQUES DE PCR

### 8.1. Analyses de l'ADNr 16S et de gène hSD65 de la souche M. austroafricanum I-2562

Afin d'identifier la nouvelle souche I-3401, une amplification par PCR de l'ADNr 16S a été réalisée en utilisant le couple d'amorces Bott 1 forward / Bott 2 reverse (voir tableau de l'exemple 5). Le produit d'amplification a été purifié en utilisant le kit Qiagen (Qiagen, Mississauga, Ontario, Canada) et le séquençage nucléotidique a été réalisé en utilisant l'amorce 244 (voir tableau de l'exemple 5). L'amplification par PCR du gène *hsp65* a été réalisée en utilisant l'amorce forward Tb 11 et l'amorce reverse Tb12 (voir tableau de l'exemple 5). Le produit d'amplification a été purifié avec le kit Qiagen et le séquençage a été effectué en utilisant l'amorce Tb11. La réaction de séquençage est effectuée avec le kit de séquençage BigDye cycle terminator (Version 3.1, Applied Biosystems, Foster City, CA, USA) comme décrit par le fabricant en utilisant 25 ng d'ADN purifié et 15 µmol des amorces universelles utilisées pour le séquençage de l'ADNr 16S des Eubactéries. La réaction est programmée comme suit : 25 cycles de 10 sec à 96°C, 5 sec à 50°C, et 4 min à 60°C. Les produits de séquençage sont purifiés sur des colonnes Centri-Sep comme décrit par le fabricant (Princeton Separations, Inc., Adelphia, NJ, USA) pour éliminer l'excès de terminateurs. Les séquençages sont réalisés sur un séquenceur automatisé à fluorescence ABT Prism 377 (Applied Biosystems, Foster City, CA). Les séquences nucléotidiques obtenues sont comparées à celles contenues dans la banque de données de gènes EMBL/GenBank et les identités sont évaluées en utilisant le système d'alignement BLAST (Altschul et al., 1997).

### 8.2. Création d'amorces et conditions spécifiques de PCR

Les séquences d'ADNr 16S de *M. austroafricanum* I-2562 et d'espèces proches des mycobactéries sont alignées et les séquences conservées et variables sont comparées. Sur la base des analyses de ces alignements multiples, plusieurs paires d'amorces spécifiques de l'espèce *M. austroafricanum* sont comparées aux séquences d'ADNr 16S disponibles en utilisant le programme de recherche dans les banques de données BLAST (Altschul et al. Nucleic Acids Res., 1997, 25 : 3389-3402). Ces séquences ont aussi été analysées afin de déterminer leur température de dénaturation (Tm), la possibilité de formation de dimères et leur contenu en (G+C) en utilisant le logiciel Amplify. La paire d'amorces la plus efficace était : MaFV2 forward et MaRV6 reverse (voir tableau de l'exemple 5). La taille du produit de PCR attendue est de 331 pb.

Les PCR sont réalisées de la façon suivante : chaque tube contient 5 µL d'ADN génomique provenant d'une lyse par chauffage ("boiling lysis") pendant 10 min de colonies isolées sur boîtes fraîchement étalées avec les souches de collection, les souches de *Mycobacterium austroafricanum,* ou les échantillons provenant d'une bio barrière ensemencée avec *M. austroafricanum* I-2562, 2.5 unités d'ADN polymérase *Taq* (Amersham Pharmacia Biotech), 5 µL du tampon ADN polymérase *Taq* dilué 10X (Amersham pharmacia biotech), 25 pmol de chacune des amorces, 4 µL de désoxyribonucléotides triphosphates à 2,5 mM (à 200 µM chacun: dATP, dGTP, dCTP, et dTTP), 2 µL de MgCl₂ à 25 mM, et de l'eau distillée stérile pour avoir un volume final de 50 µL. Le contrôle négatif contient le même mélange que décrit ci-dessus sauf que l'ADN est remplacé par de l'eau stérile. Les échantillons sont préalablement chauffés 3 min à 95°C dans un appareil Bio-Rad Thermal iCycler (Bio-Rad, Mississauga, Ontario, Canada) puis la température est abaissée à 80°C avant leur addition au mélange enzymatique ADN polymérase *Taq* /tampon dilué 10X. Dans le but d'obtenir des conditions d'amplification spécifique, les conditions d'amplification sont les suivantes : 30 sec à 94°C, 1 min à 68°C, 1 min à 72°C répété pendant 30 cycles et l'extension finale est effectuée à 72°C pendant 7 min. Les tubes de PCR sont conservés à 4°C jusqu'au moment d'effectuer l'électrophorèse sur gel d'agarose. 10 µL de chacun des produits de PCR sont mélangés avec 2 µL de tampon de dépôt contenant du glycérol à 30% (v/v), du bleu de bromophénol à 0,15% (w/v), du xylène cyanol à 0,15% (w/v). Les différents produits de PCR ainsi préparés et le marqueur d'ADN 1kb GeneRuler^{™} (MBI Fermentas, Inc., Burlington, Ontario, Canada) sont déposés sur gel d'agarose à 1% (w/v) préparé dans du TAE. Après migration, le gel est coloré au bromure d'éthidium et révélé aux UVs à 254 nm sur un transilluminateur. Les gels sont photographiés en utilisant un film Polaroid de type 57.

### EXEMPLE 9: DETECTION DE LA PRESENCE DE L'ORGANISATION GENETIQUE mpd AU SEIN DE LA NOUVELLE SOUCHE M. AUSTROAFRICANUM I-3401.

La biodégradation du MTBE par la souche *M. austroafricanum* I-3401 étant similaire à celle de *M. austroafricanum* I-2562, la présence ou non du « cluster » *mpd* au sein de la nouvelle souche a été étudiée. Ainsi, des couples d'amorces, MF1/MR1, MF2/MR2, MF3/MR3 et MF4/MR4 amplifiant spécifiquement dans les différents gènes du cluster *mpd* ont été créés (Tableau de l'exemple 5). Des amplifications par PCR effectuées sur l'ADN génomique de *M. austroafricanum* I-3401 ou I-2562 (ce dernier, comme contrôle positif) ont été réalisées (Figure 11) et montrent des amplifications positives pour tous les couples d'amorces. Ceci démontre la présence d'une organisation génétique similaire à celle des gènes *mpd* de *M. austroafricanum* I-2562 au sein du génome de la nouvelle souche *M. austroafricanum* I-3401. Il est donc vraisemblable que la nouvelle souche utilise le même chemin réactionnel pour assimiler le 2-M1,2-PD au cours du catabolisme du MTBE.

### EXEMPLE 10 : DETECTION SPECIFIQUE DE M. AUSTROAFRICANUM 1-2562 ET I-3401 PAR PCR.

Les amorces MaFV2 et MaRV6 spécifiques de l'espèce *M*. *austroafricanum* ont été créées (voir tableau de l'exemple 5). La spécificité de ces amorces se fixant dans deux des régions variables V2 et V6 de l'ADNr 16S de *M*. *austroafricanum* I-2562 a été évaluée en utilisant de l'ADN génomique de souches plus ou moins proches. Les résultats présentés sur la Figure 12 montrent que seuls les ADN génomique des deux souches de *M. austroafricanum,* I-2562 et I-3401, métabolisant le MTBE permet d'obtenir une amplification PCR positive. Ces amorces permettent donc de détecter spécifiquement les microorganismes de cette espèce. D'autres microorganismes du genre *Nocardiaceae* ne présentent pas d'amplification par PCR.

### EXEMPLE 11 : COMPARAISON ENTRE LES SOUCHES I-2562 ET I-3401

| | *M. ausfroafricanum* | |
|---|---|---|
| | I-2562 | I-3401 |
| Capacité de dégradation de l'ETBE (130 mg/L) | 100 jours | **33** jours |
| Métabolisme du MTBE : assimilation des intermédiaires | - | **accumulation** du 2-M 1,2 PD |
| Vitesse de dégradation du 2-méthyl 1,2 propanediol | **413** mg.g⁻¹ poids sec.h⁻¹ | 60 mg.g⁻¹ poids sec.h⁻¹ |

Une différence importante entre la souche I-2562 et la souche I-3401 réside dans leur capacité de dégradation de l'ETBE : pour une concentration équivalente d'ETBE (130 mg/L), M. austroafricanum I-2562 dégrade l'ETBE en 100 jours alors que I-3401 réalise cette dégradation trois fois plus vite. D'autre part, la vitesse de dégradation du 2-M1,2-PD quand il est utilisé directement comme substrat est environ 7 fois plus rapide chez I-2562 que I-3401. Enfin, il est possible de détecter la présence de 2-M1,2-PD au cours de la dégradation du tert-butyl alcool (TBA) chez I-3401 du fait de cette différence de vitesse de dégradation du 2-M1,2-PD.

## Revendications

1. Polypeptide isolé ou purifié ayant une activité dans la voie de dégradation du MTBE, et/ou l'un au moins des intermédiaires cataboliques du MTBE, préférentiellement sélectionné dans le groupe consistant en le *tert*-butyl alcool (TBA), le 2-méthyl 1,2-propanediol (2-M1,2-PD), l'hydroxyisobutyraldéhyde, l'acide hydroxyisobutyrique (HIBA), ledit polypeptide étant sélectionné dans le groupe consistant en :
a) un polypeptide comprenant une séquence en acides aminés sélectionnée dans le groupe consistant en SEQ ID NO :2 ou SEQ ID NO :10,
b) un polypeptide comprenant une séquence en acides aminés qui présente au moins 70 % d'identité, préférentiellement 75%, 80%, 90%, 95%, 98% ou 99% d'identité avec la séquence en acides aminés d'un polypeptide comprenant SEQ ID NO :2, le polypeptide tel que défini en a) ou b) de séquence SEQ ID NO :2 ayant une activité de déshydrogénation de l'hydroxyisobutyraldéhyde en HIBA, et
le polypeptide tel que défini en a) de séquence SEQ ID NO :10 ayant une activité de régulateur transcriptionnel

2. Acide nucléique purifié ou isolé, **caractérisé en ce qu'**il code au moins un polypeptide selon la revendication 1.

3. Acide nucléique isolé ou purifié codant au moins un polypeptide ayant une activité dans la voie de dégradation du MTBE, ou l'un au moins des intermédiaires cataboliques du MTBE, préférentiellement le TBA, le 2 M1,2-PD, l'hydroxyisobutyraldéhyde, le HIBA,, sélectionné dans le groupe consistant en :
e) un acide nucléique comprenant au moins l'une quelconque des séquences nucléotidiques sélectionnée dans le groupe consistant en SEQ ID NO:1 ou SEQ ID NO:9, ou complément de celle-ci,
f) un acide nucléique comprenant au moins une séquence nucléotidique qui présente au moins 70 % d'identité, préférentiellement 75%, 80%, 90%, 95%, 98% ou 99% d'identité avec la séquence nucléotidique d'un acide nucléique tel que défini en e).

4. Acide nucléique, **caractérisé en ce qu'**il comprend la séquence nucléotidique SEQ ID NO :11.

5. Acide nucléique selon l'une quelconque des revendications 2 à 4, **caractérisé en ce que** sa transcription est sous le contrôle d'un promoteur unique.

6. Acide nucléique selon l'une quelconque des revendications 2 à 5, **caractérisé en ce que** sa transcription est induite par la présence de MTBE, et/ou l'un au moins des intermédiaires cataboliques du MTBE, préférentiellement sélectionné dans le groupe consistant en le TBA, le 2-M1,2-PD, l'hydroxyisobutyraldéhyde et le HIBA.

7. Acide nucléique selon la revendication 6, **caractérisé en ce qu'**il est organisé en opéron.

8. Polypeptide selon la revendication 1 ou acide nucléique selon l'une quelconque des revendications 2 à 7, **caractérisé en ce qu'**il est isolé et/ou purifié d'une souche bactérienne choisie parmi les souches *Mycobacterium austroafricanum* enregistrées dans la collection CNCM sous les numéros I-3401 et I-2562.

9. Acide nucléique recombinant, **caractérisé en ce qu'**il comprend un acide nucléique selon l'une quelconque des revendications 2 à 8.

10. Vecteur **caractérisé en ce qu'**il comprend un acide nucléique selon l'une quelconque des revendications 2 à 9.

11. Cellule hôte isolée comprenant soit au moins l'un des acides nucléiques selon l'une quelconque des revendications 2 à 9, soit au moins l'un des vecteurs selon la revendication 10.

12. Procédé d'identification d'une cellule ou d'un acide nucléique d'une cellule susceptible de dégrader du MTBE et/ou au moins l'un des intermédiaires cataboliques du MTBE, préférentiellement sélectionné dans le groupe consistant en le TBA, le 2-M1,2-PD, l'hydroxyisobutyraldéhyde et le HIBA, comprenant :
- optionnellement une étape d'ensemencement de la cellule sur un milieu supplémenté en MTBE et/ou au moins l'un des intermédiaires cataboliques du MTBE, préférentiellement sélectionné dans le groupe consistant en le TBA, le 2-M1,2-PD, l'hydroxyisobutyraldéhyde et le HIBA,
- une étape de criblage des acides nucléiques de ladite cellule par hybridation avec au moins une sonde, ladite sonde comprenant la séquence nucléotidique complémentaire d'un fragment d'au moins 50 nucléotides consécutifs de la séquence nucléotidique sélectionnée dans le groupe consistant en SEQ ID NO :1 ou SEQ ID NO :9, ou ladite sonde comprenant un fragment du gène mpdB de 591 paires de bases ayant pour séquence la séquence SEQ ID n°: 14.

13. Souche bactérienne *Mycobacterium austroafricanum* enregistrées dans la collection CNCM sous le numéro I-3401.

14. Utilisation du polypeptide SEQ ID NO :2 selon la revendication 1 pour la déshydrogénation de l'hydroxyisobutyraldéhyde en HIBA.

15. Procédé de traitement d'effluents aqueux comprenant du MTBE, du ETBE et/ou au moins l'un des intermédiaires cataboliques du MTBE, préférentiellement sélectionné dans le groupe consistant en le TBA, le 2-M1,2-PD, l'hydroxyisobutyraldéhyde, le HIBA, afin de réduire leur concentration, **caractérisé en ce qu'**il comprend l'ensemencement dudit effluent aqueux par au moins une cellule selon l'une quelconque des revendications 11 ou 13 ou identifiée par le procédé selon la revendication 12.

## Claims

1. Isolated or purified polypeptide with an activity in the degradation pathway of MTBE and/or at least one of the catabolites of MTBE, preferably selected from the group consisting of tert-butyl alcohol (TBA), 2-methyl-1,2-propanediol (2-M-1,2-PD), hydroxyisobutyraldehyde, hydroxyisobutyric acid (HIBA), said polypeptide being selected from the group consisting of:
a) a polypeptide comprising an amino acid sequence selected from the group consisting of SEQ ID No: 2 or SEQ ID No: 10,
b) a polypeptide comprising an amino acid sequence with at least 70% identity, preferably 75%, 80%, 90%, 95%, 98% or 99% identity, with the amino acid sequence of a polypeptide comprising SEQ ID NO:2, the polypeptide as defined in a) or b) of SEQ ID NO:2 having a dehydrogenation activity from hydroxyisobutyraldehyde into HIBA, and
the polypeptide as defined in a) of SEQ ID NO: 10 having a transcriptional regulator activity.

2. Purified or isolated nucleic acid, **characterized in that** it codes for at least one polypeptide as claimed in claim 1.

3. Isolated or purified nucleic acid coding for at least one polypeptide with an activity in the degradation pathway of MTBE or at least one catabolite of MTBE, preferably TBA, 2-M-1,2-PD, hydroxyisobutyraldehyde, HIBA, selected from the group consisting of:
e) a nucleic acid comprising at least any one of the nucleotide sequences selected from the group consisting of SEQ ID No: 1 or SEQ ID No: 9, or its complement,
f) a nucleic acid comprising at least one nucleotide sequence with at least 70% identity, preferably 75%, 80%, 90%, 95%, 98% or 99% identity, with the nucleotide sequence of a nucleic acid as defined in e).

4. Nucleic acid, **characterized in that** it comprises the nucleotide sequence SEQ ID NO: 11.

5. Nucleic acid as claimed in any one of claims 2 to 4, **characterized in that** its transcription is under the control of a single promoter.

6. Nucleic acid as claimed in any one of claims 2 to 5, **characterized in that** its transcription is induced by the presence of MTBE and/or at least one of the catabolites of MTBE, preferably selected from the group consisting of TBA, 2-M-1,2-PD, hydroxyisobutyraldehyde and HIBA.

7. Nucleic acid as claimed in claim 6, **characterized in that** it is organized as an operon.

8. Polypeptide as claimed in claim 1, or nucleic acid as claimed in any one of claims 2 to 7, **characterized in that** it is isolated and/or purified from a bacterial strain chosen from the *Mycobacterium austroafricanum* strains deposited at the CNCM collection as numbers I-3401 and I-2562.

9. Recombinant nucleic acid, **characterized in that** it comprises a nucleic acid as claimed in any one of claims 2 to 8.

10. Vector **characterized in that** it comprises a nucleic acid as claimed in any one of claims 2 to 9.

11. Isolated host cell comprising either at least one of the nucleic acids as claimed in any one of claims 2 to 9, or at least one of the vectors according to claim 10.

12. A method of identifying a cell or a nucleic acid of a cell capable of degrading MTBE and/or at least one of the catabolites of MTBE, preferably selected from the group consisting of TBA, 2-M-1,2-PD, hydroxyisobutyraldehyde, HIBA, which comprises:
- optionally a step of seeding the cell on a medium which is supplemented with MTBE and/or at least one of the catabolites of MTBE, preferably selected from the group consisting of TBA, 2-M-1,2-PD, hydroxyisobutyraldehyde, HIBA,
- a step of screening by hybridization the nucleic acids of said cell with at least one probe, said probe comprising the nucleotide sequence which is complementary to a fragment of at least 50 consecutive nucleotides of the nucleotide sequence selected from the group consisting of SEQ ID No: 1 or SEQ ID No: 9, or said probe comprising a fragment of the mpdB gene of 591 base pairs whose sequence is the sequence SEQ ID No: 14.

13. A bacterial *Mycobacterium austroafricanum* strain deposited in the CNCM collection as number I-3401.

14. Use of the polypeptide SEQ ID NO:2 according to claim 1 for the dehydrogenation from hydroxyisobutyraldehyde into HIBA.

15. A method of treating waste water comprising MTBE, ETBE and/or at least one of the catabolites of MTBE, preferably selected from the group consisting of TBA, 2-M-1,2-PD, hydroxyisobutyraldehyde, HIBA, in order to reduce their concentration, **characterized in that** it comprises seeding said waste water with at least one cell as claimed in any one of claims 11 or 13 or identified according to claim 12.

## Patentansprüche

1. Isoliertes oder gereinigtes Polypeptid, welches eine Aktivität im Abbauweg von MTBE und/oder wenigstens einem der Abbauzwischenprodukte von MTBE, welches bevorzugt ausgewählt ist aus der Gruppe bestehend aus tert-Butylalkohol (TBA), 2-Methyl-1,2-propandiol (2-M1,2-PD), Hydroxyisobutyraldehyd und Hydroxyisobuttersäure (HIBA), aufweist, wobei das Polypeptid ausgewählt ist aus der Gruppe bestehend aus:
a) einem Polypeptid, welches eine Aminosäuresequenz umfasst, die ausgewählt ist aus der Gruppe bestehend aus SEQ ID NO:2 oder SEQ ID NO: 10,
b) einem Polypeptid, welches eine Aminosäuresequenz umfasst, die wenigstens 70 % Identität, bevorzugt 75 %, 80 %, 90 %, 95 %, 98 % oder 99 % Identität, mit der Aminosäuresequenz eines Polypeptids, welches die SEQ ID NO: 2 umfasst, aufweist,
wobei das in a) oder b) definierte Polypeptid der Sequenz SEQ ID NO: 2 eine Aktivität der Dehydrierung von Hydroxyisobutyraldehyd zu HIBA aufweist, und wobei
das in a) definierte Polypeptid der Sequenz SEQ ID NO: 10 eine Aktivität eines Transkriptionsregulators aufweist.

2. Gereinigte oder isolierte Nukleinsäure, **dadurch gekennzeichnet, dass** sie wenigstens ein Polypeptid gemäß Anspruch 1 kodiert.

3. Isolierte oder gereinigte Nukleinsäure, welche wenigstens ein Polypeptid kodiert, das eine Aktivität im Abbauweg von MTBE oder wenigstens einem der Abbauzwischenprodukte von MTBE, bevorzugt TBA, 2-M1,2-PD, Hydroxyisobutyraldehyd, HIBA, aufweist und ausgewählt ist aus der Gruppe bestehend aus:
e) einer Nukleinsäure, welche wenigstens irgendeine der Nukleotidsequenzen umfasst, die ausgewählt sind aus der Gruppe bestehend aus SEQ ID NO: 1 oder SEQ ID NO: 9 oder deren komplementären Sequenzen,
f) einer Nukleinsäure, welche wenigstens eine Nukleotidsequenz umfasst, die wenigstens 70 % Identität, bevorzugt 75 %, 80 %, 90 %, 95 %, 98 % oder 99 % Identität, mit der Nukleotidsequenz einer in e) definierten Nukleinsäure aufweist.

4. Nukleinsäure, **dadurch gekennzeichnet, dass** sie die Nukleotidsequenz SEQ ID NO: 11 umfasst.

5. Nukleinsäure gemäß irgendeinem der Ansprüche 2 bis 4, **dadurch gekennzeichnet, dass** deren Transkription unter der Kontrolle eines Einzelpromotors steht.

6. Nukleinsäure gemäß irgendeinem der Ansprüche 2 bis 5, **dadurch gekennzeichnet, dass** ihre Transkription durch die Anwesenheit von MTBE und/oder wenigstens einem der Abbauzwischenprodukte von MTBE, welches bevorzugt ausgewählt ist aus der Gruppe bestehend aus TBA, 2-M1,2-PD, Hydroxyisobutyraldehyd und HIBA, induziert wird.

7. Nukleinsäure gemäß Anspruch 6, **dadurch gekennzeichnet, dass** sie in einem Operon organisiert ist.

8. Polypeptid gemäß Anspruch 1 oder Nukleinsäure gemäß irgendeinem der Ansprüche 2 bis 7, **dadurch gekennzeichnet, dass** es/sie aus einem Bakterienstamm isoliert und/oder aufgereinigt ist, der ausgewählt ist aus den *Mycobacterium austroafricanum*-Stämmen, die in der Sammlung CNCM unter den Nummern I-3401 und I-2562 registriert sind.

9. Rekombinante Nukleinsäure, **dadurch gekennzeichnet, dass** sie eine Nukleinsäure gemäß irgendeinem der Ansprüche 2 bis 8 umfasst.

10. Vektor, **dadurch gekennzeichnet, dass** er eine Nukleinsäure gemäß irgendeinem der Ansprüche 2 bis 9 umfasst.

11. Isolierte Wirtszelle, welche entweder wenigstens eine der Nukleinsäuren gemäß irgendeinem der Ansprüche 2 bis 9 oder wenigstens einen Vektor gemäß Anspruch 10 umfasst.

12. Verfahren zur Identifizierung einer Zelle oder Nukleinsäure einer Zelle, welche MTBE und/oder wenigstens eines der Abbauzwischenprodukte von MTBE, bevorzugt ausgewählt aus der Gruppe bestehend aus TBA, 2-M1,2-PD, Hydroxyisobutyraldehyd und HIBA, abbauen kann, umfassend:
- optional einen Schritt, in dem die Zelle auf einem Medium, welches mit MTBE und/oder wenigstens einem der Abbauzwischenprodukte von MTBE, bevorzugt ausgewählt aus der Gruppe bestehend aus TBA, 2-M1,2-PD, Hydroxyisobutyraldehyd und HIBA, supplementiert ist, ausgesät wird,
- einen Screening-Schritt, der Nukleinsäuren der Zelle durch Hybridisierung mit wenigstens einer Sonde getrennt werden, wobei die Sonde eine Nukleotidsequenz umfasst, die komplementär zu einem Fragment von wenigstens 50 aufeinander folgenden Nukleotiden der Nukleotidsequenz, ausgewählt aus der Gruppe bestehend aus SEQ ID NO: 1 oder SEQ ID NO: 9, ist, oder wobei die Sonde ein Fragment des Gens mpdB von 591 Basenpaaren, welches die SEQ ID NO: 14 als Sequenz aufweist, umfasst.

13. Bakterienstamm *Mycobacterium austroafricanum,* welcher in der Sammlung CNCM unter der Nummer I-3401 registriert ist.

14. Verwendung des Polypeptids SEQ ID NO: 2 gemäß Anspruch 1 zur Dehydrierung von Hydroxyisobutyraldehyd zu HIBA.

15. Verfahren zur Behandlung von wässrigen Abwässern, welche MTBE, ETBE und/oder wenigstens eines der Abbauzwischenprodukte von MTBE, bevorzugt ausgewählt aus der Gruppe bestehend aus TBA, 2-M1,2-PD, Hydroxyisobutyraldehyd und HIBA, enthalten, zur Reduzierung ihrer Konzentration, **dadurch gekennzeichnet, dass** das Verfahren das Animpfen des wässrigen Abwassers mit wenigstens einer Zelle gemäß irgendeinem der Ansprüche 11 oder 13 oder mit einer durch das Verfahren von Anspruch 12 identifizierten Zelle umfasst.
